# EUROPÄISCHE PATENTSCHRIFT

(11) **EP 4 093 725 B1**
(45) Veröffentlichungstag und Bekanntmachung des Hinweises auf die Patenterteilung: **31.01.2024**
(21) Anmeldenummer: 20812054.3
(22) Anmeldetag: 30.11.2020
(51) Int. Cl.: C07C 29/70, C07C 31/30

(54) **VERFAHREN ZUR ENERGIEEFFIZIENTEN HERSTELLUNG VON NATRIUM- UND KALIUMALKOHOLATEN**
METHOD FOR THE ENERGY-EFFICIENT PREPARATION OF SODIUM AND POTASSIUM ALCOHOLATES
PROCÉDÉ DE FABRICATION À HAUT RENDEMENT ÉNERGÉTIQUE D'ALCOOLS DE SODIUM ET DE POTASSIUM

(30) Priorität: 23.01.2020 EP 20153356
(43) Veröffentlichungstag der Anmeldung: 30.11.2022
(73) Patentinhaber: Evonik Operations GmbH, 45128 Essen (DE)
(72) Erfinder: ROETTGER, Dirk, 50672 Köln (DE); REIMANN, Sebastian, 50389 Wesseling (DE); NEUMANN, Manfred, 45770 Marl (DE); SCHRÖDER, Moritz, 48153 Münster (DE); ZITZEWITZ, Philip, 45721 Haltern am See (DE); PAUL, Niklas, 45770 Marl (DE); RIX, Armin Matthias, 45770 Marl (DE)
(74) Vertreter: Evonik Patent Association
(86) Internationale Anmeldenummer: PCT/EP2020/083892
(87) Internationale Veröffentlichungsnummer: WO 2021/148175

(56) Entgegenhaltungen:
- WO-A1-2010/097318
- US-A- 4 566 947

## Beschreibung

Die vorliegende Erfindung betrifft ein Verfahren zur Herstellung von Alkalimetallalkoholaten im Gegenstrom durch Reaktivrektifikation, wobei das Alkalimetall aus Natrium und Kalium ausgewählt ist. Dabei wird der Alkohol in einem ersten Schritt im Gegenstrom mit dem betreffenden Alkalimetallhydroxid umgesetzt. Die erhaltene Mischung aus Alkohol und Wasser wird in einem zweiten Schritt in einer Rektifikationskolonne aufgetrennt und der anfallende alkoholische Brüden verdichtet, wodurch sich dessen Temperatur erhöht. Die Energie, die beim Kühlen des verdichteten Brüden abgeführt wird, wird im Unterschied zu den herkömmlichen Verfahren dadurch genutzt, dass sie im ersten Schritt des erfindungsgemäßen Verfahrens eingesetzt wird. Dies ermöglicht eine energieeffiziente Herstellung der betreffenden Alkalimetallalkoholate.

### 1. Hintergrund der Erfindung

Die Herstellung von Alkalimetallalkoholaten ist ein wichtiger industrieller Prozess.

Alkalimetallalkoholate werden als starke Basen in der Synthese zahlreicher Chemikalien, z.B. bei der Herstellung von Pharma- oder Agrowirkstoffen, eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion <1> entstehende Reaktionswasser mit dem Destillat entfernt wird.

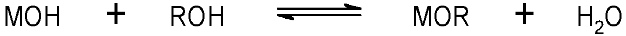

Ein solches Verfahrensprinzip ist beispielsweise in der US 2,877,274 A beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der WO 01/42178 A1 erneut beschrieben.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben. Das Schleppmittel dient hierbei zur Trennung von Wasser und dem wasserlöslichen Alkohol. Bei beiden Patentschriften wird das Kondensat einer Phasentrennung unterzogen, um das Reaktionswasser abzutrennen.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1.

Bei den im Stand der Technik beschriebenen Synthesen der Alkalimetallalkoholate durch Reaktivrektifikation werden üblicherweise Brüden erhalten, welche den eingesetzten Alkohol und Wasser umfassen. Es ist aus wirtschaftlichen Gründen sinnvoll, den von den Brüden umfassten Alkohol wieder in der Reaktivdestillation als Edukt einzusetzen. Deshalb werden die Brüden üblicherweise einer Rektifikationskolonne zugeführt und der darin enthaltene Alkohol abgetrennt (beschrieben beispielsweise in der GB 737 453 A und der US 4,566,947 A). Der so zurückgewonnene Alkohol wird dann beispielsweise als Edukt der Reaktivdestillation zugeführt. Alternativ oder zusätzlich kann ein Teil des Alkoholbrüden zur Beheizung der Rektifikationskolonne genutzt werden (beschrieben in WO 2010/097318 A1). Dazu muss der Brüden allerdings verdichtet werden, um das zur Beheizung der Rektifikationskolonne nötige Temperaturniveau zu erreichen. Insbesondere eine mehrstufige Verdichtung des Brüden ist thermodynamisch vorteilhaft. Hierbei wird der Brüden zwischen den Verdichtungsstufen gekühlt. Darüber hinaus trägt die Zwischenkühlung dazu bei, die maximal zulässige Temperatur des Verdichters nicht zu überschreiten.

Der Nachteil dieser in den herkömmlichen Verfahren durchgeführten Kühlung ist, dass die dabei entzogene Energie ungenutzt dissipiert.

Es besteht demnach der Bedarf nach verbesserten Verfahren zur Herstellung von Alkoholaten des Natriums und Kaliums durch Reaktivdestillation. Diese sollen die vorgenannten Nachteile vermeiden und insbesondere eine energieeffiziente Nutzung der bei der Verdichtung und Abkühlung der Brüden anfallenden Wärme ermöglichen.

### 2. Kurzzusammenfassung der Erfindung

Die vorliegende Erfindung betrifft demnach ein Verfahren zur Herstellung mindestens eines Alkalimetallalkoholats der Formel M_{A}OR, wobei R ein C₁ bis C₆-Kohlenwasserstoffrest, bevorzugt Methyl, ist, und wobei M_{A} ein Metall ausgewählt aus Natrium, Kalium, bevorzugt Natrium ist, wobei:
(a1) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend ROH und M_{A}OR entnommen wird und am oberen Ende von **RR_{A}** ein Brüdenstrom **S_{AB}** umfassend Wasser und ROH entnommen wird,
(a2) und gegebenenfalls, gleichzeitig mit und räumlich getrennt von Schritt (a1), ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohprodukt **RP_{B}** umfassend M_{B}OR, Wasser, ROH, M_{B}OH umgesetzt wird, wobei M_{B} ein Metall ausgewählt aus Natrium, Kalium, bevorzugt Kalium, ist,
wobei am unteren Ende von **RR_{B}** ein Sumpfproduktstrom **S_{BP}** umfassend ROH und M_{B}OR entnommen wird und am oberen Ende von **RR_{B}** ein Brüdenstrom **S_{BB}** umfassend Wasser und ROH entnommen wird,
(b) der Brüdenstrom **S_{AB},** und, wenn Schritt (a2) durchgeführt wird, der Brüdenstrom **S_{BB},** vermischt mit **S_{AB}** oder getrennt von **S_{AB},** bevorzugt vermischt mit **S_{AB},** in eine Rektifikationskolonne **RD_{A}** geleitet wird, und in **RD_{A}** in einen ROH umfassenden Brüdenstrom **S_{B2}** mit einem Druck **p_{B2}** und einer Temperatur **T_{B2}** am Kopf von **RD_{A}** und einen Wasserstrom **S_{W}** am Sumpf von **RD_{A}** aufgetrennt wird,
(c) mindestens ein Teil des Brüdenstroms **S_{B2}** verdichtet wird, wodurch ein gegenüber **S_{B2}** verdichteter Brüdenstrom **S_{B3}** mit einem Druck **p_{B3}** > **p_{B2}** und einer Temperatur **T_{B3}** > **T_{B2}** erhalten wird,
(d) Energie von **S_{B3}** auf mindestens einen von **RP_{A}, S_{AE1}, S_{AE2}, S_{AP}, S_{AB}** übertragen wird, und, wenn Schritt (a2) durchgeführt wird, alternativ oder zusätzlich, bevorzugt zusätzlich, auf mindestens einen von **RP_{B}, S_{BE1}, S_{BE2}, S_{BP}, S_{BB}** übertragen wird,
wodurch ein Brüdenstrom **S_{B4}** mit einem geringeren Energiegehalt als **S_{B3}** erhalten wird.

In einer bevorzugten Ausführungsform des erfindungsgemäßen Verfahrens wird in einem Schritt (e) der Brüdenstrom **S_{B4}** zur Beheizung der Rektifikationskolonne **RD_{A}** eingesetzt und/oder, bevorzugt und, in diese zurückgeführt.

### 3. Abbildungen

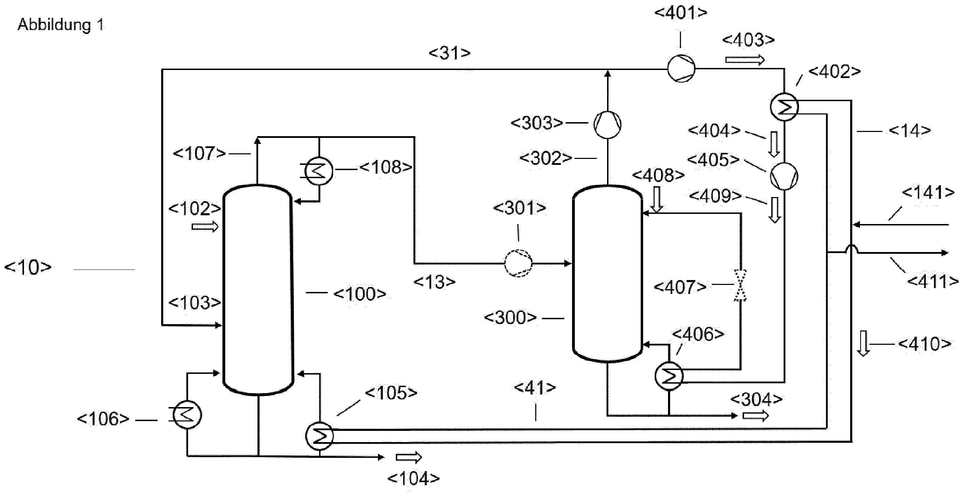

Abbildung 1 zeigt eine Ausführungsform des erfindungsgemäßen Verfahrens, in welcher NaOH **S_{AE2}** <102> in einer Reaktionskolonne **RR_{A}** <100> mit Methanol **S_{AE1}** <103> zu Natriummethanolat <104> umgesetzt wird. Am Sumpf der Reaktionskolonne wird ein Methanol-Methanolat-Gemisch **S_{AP*}** <104> entnommen. Der erhaltene Brüden **S_{AB}** <107> wird einer Wasser/Methanol-Kolonne **RD_{A}** <300> zugeführt, in welcher Methanol als Brüden **S_{B2}** <302> destillativ rückgewonnen wird. Der Brüden **S_{B2}** <302> wird mittels Verdichter **VD_{AB2}** <303> vorverdichtet und im Verdichter **VD₁** <401> zum Brüden **S_{B3}** <403> verdichtet. Die nach der Verdichtung des Brüden **S_{B3}** <403> im Zwischenkühler **WT_{X}** <402> abgeführte Energie wird dem Verdampfer **V_{SA}** <105> am Sumpf der Reaktionskolonne **RR_{A}** <100> zugeführt. Nach Abführung der Energie wird der erhaltene Brüden **S_{B4}** <404> optional erneut mittels Verdichter **VDₓ** <405> verdichtet und der dann erhaltene Brüden **S_{B5}** <409> dem Verdampfer **V_{SRD}** <406> am Sumpf der Rektifikationskolonne **RD_{A}** <300> zur Beheizung zugeführt.

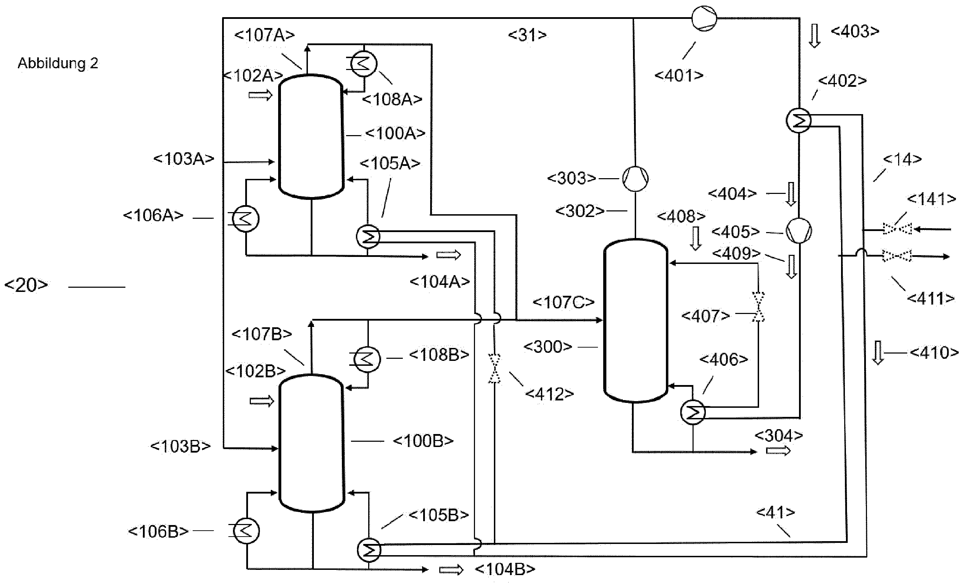

Abbildung 2 zeigt eine Ausführungsform des erfindungsgemäßen Verfahrens, in welcher NaOH **S_{AE2}** <102A>und KOH **S_{BE2}** <102B> jeweils in zwei getrennten Reaktionskolonnen **RR_{A}** <100A>, **RR_{B}** <100B> mit Methanol **S_{AE2}**<103A> bzw. **S_{BE2} <103B>** zum jeweiligen Methanolat umgesetzt werden. Am Sumpf der Reaktionskolonnen wird jeweils das Methanol-Methanolat-Gemisch **S_{AP*}** <104A> bzw. **S_{BP*<}104B>** entnommen. Die erhaltenen Brüden werden als Mischung <107C> einer Wasser/Methanol-Kolonne **RD_{A}** <300> zugeführt, in welcher Methanol als Brüden **S_{B2}** <302> destillativ rückgewonnen wird. Dieses wird mittels Verdichter **VD_{AB2}** <303> vorverdichtet uund mittels Verdichter **VD₁**<401 > zum verdichteten Brüden **S_{B3}** <403> verdichtet. Die aus dem verdichteten Brüden **S_{B3}** <403> im Zwischenkühler **WT_{X}** <402> abgeführte Energie wird dem Verdampfer **V_{SA}** <105A> am Sumpf der Reaktionskolonne **RR_{A}** <100A> und dem Verdampfer **V_{SB}** <105B> am Sumpf der Reaktionskolonne **RR_{B}** <100B> zugeführt. Nach dem Zwischenkühler **WT_{X}** <402> passiert der erhaltene Brüden **S_{B4}** <404> optional einen weiteren Verdichter **VDₓ** <405> und wird als Brüden **S_{B5}** <409> zur Beheizung dem Verdampfer **V_{SRD}** <406> am Sumpf der Rektifikationskolonne **RD_{A}** <300> zugeführt.

### 4. Detaillierte Beschreibung der Erfindung

### 4.1 Schritt (a1) des erfindungsgemäßen Verfahrens

Im Schritt (a1) des erfindungsgemäßen Verfahrens wird ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt.

Als "Reaktivrektifikationskolonne" wird erfindungsgemäß eine Rektifikationskolonne definiert, in der zumindest in einigen Teilen die Umsetzung nach Schritt (a1) oder Schritt (a2) des erfindungsgemäßen Verfahrens ablaufen. Sie kann auch als "Reaktionskolonne" abgekürzt werden.

In Schritt (a1) des erfindungsgemäßen Verfahrens wird am unteren Ende von **RR_{A}** ein Sumpfproduktstrom **S_{AP}** umfassend ROH und M_{A}OR entnommen. Am oberen Ende von **RR_{A}** wird ein Brüdenstrom **S_{AB}** umfassend Wasser und ROH entnommen.

R ist im erfindungsgemäßen Verfahren ein C₁ bis C₆-Kohlenwasserstoffrest, bevorzugt ausgewählt aus der Guppe bestehend aus Methyl, Ethyl, *n*-Propyl, *iso*-Propyl, *n*-Butyl, *sec*-Butyl, *iso*-Butyl, *tert*-Butyl, Isomeren des Pentyls, wie beispielsweise *n*-Pentyl, bevorzugter ausgewählt aus der Gruppe bestehend aus Methyl, Ethyl, *n*-Propyl, *iso*-Propyl, noch bevorzugter ausgewählt aus der Gruppe bestehend aus Methyl, Ethyl. Besonders bevorzugt ist R Methyl und ROH demnach Methanol.

M_{A} ist ausgewählt aus Natrium, Kalium, und bevorzugt Natrium.

Der Eduktstrom **S_{AE1}** umfasst ROH. In einer bevorzugten Ausführungsform liegt der Massenanteil von ROH in **S_{AE1}** bei ≥ 95 Gew.-%, noch bevorzugter bei ≥ 99 Gew.-%, wobei **S_{AE1}** ansonsten insbesondere Wasser aufweist.

Der in Schritt (a1) des erfindungsgemäßen Verfahrens als Eduktstrom **S_{AE1}** eingesetzte Alkohol ROH kann auch handelsüblicher Alkohol mit einem Alkoholmassenanteil von mehr als 99.8 Gew.-% und einem Massenanteil an Wasser von bis zu 0.2 Gew.-% sein.

Der Eduktstrom **S_{AE1}** wird bevorzugt dampfförmig zugegeben.

Der Eduktstrom **S_{AE2}** umfasst M_{A}OH. In einer bevorzugten Ausführungsform umfasst **S_{AE2}** neben M_{A}OH mindestens eine weitere Verbindung ausgewählt aus Wasser, ROH. Noch bevorzugter umfasst **S_{AE2}** neben M_{A}OH Wasser, dann handelt es sich bei **S_{AE2}** um eine wässrige Lösung von M_{A}OH.

Wenn der Eduktstrom **S_{AE2}** M_{A}OH und Wasser umfasst, liegt der Massenanteil von M_{A}OH, bezogen auf das Gesamtgewicht der wässrigen Lösung, welche **S_{AE2}** bildet, insbesondere im Bereich von 10 bis 55 Gew.-%, bevorzugt von 15 bis 54 Gew.-%, bevorzugter von 30 bis 53 Gew.-% und besonders bevorzugt von 45 bis 52 Gew.-%. Am bevorzugtesten liegt der Anteil von M_{A}OH in der wässrigen Lösung dann bei 50 Gew.-%.

Wenn der Eduktstrom **S_{AE2}** M_{A}OH und ROH umfasst, liegt der Massenanteil von M_{A}OH in ROH, bezogen auf das Gesamtgewicht der Lösung, welche **S_{AE2}** bildet, insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-%.

In dem besonderen Fall, in dem der Eduktstrom **S_{AE2}** neben M_{A}OH sowohl Wasser als auch ROH umfasst, ist es besonders bevorzugt, dass der Massenanteil von M_{A}OH in ROH und Wasser, bezogen auf das Gesamtgewicht der Lösung, welche **S_{AE2}** bildet, insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-% liegt.

Schritt (a1) des erfindungsgemäßen Verfahrens wird in einer Reaktivrektifikationskolonne (oder "Reaktionskolonne") **RR_{A}** durchgeführt.

Schritt (a2) des erfindungsgemäßen Verfahrens wird in einer Reaktivrektifikationskolonne (oder "Reaktionskolonne") **RR_{B}** durchgeführt.

Bevorzugt enthält die Reaktionskolonne **RR_{A}** bzw. **RR_{B}** Einbauten. Geeignete Einbauten sind zum Beispiel Böden, strukturierte Packungen oder unstrukturierte Packungen. Wenn die Reaktionskolonne **RR_{A}** bzw. **RR_{B}** Böden enthält, so sind Glockenböden, Ventilböden, Tunnelböden, Thormann-Böden, Kreuzschlitzglockenböden oder Siebböden geeignet. Wenn die Reaktionskolonne **RR_{A}** bzw. **RR_{B}** Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt. Bei dieser Ausführungsform darf zudem in allen Teilen des Kolonnenquerschnitts, die mehr als 2 % des gesamten Kolonnenquerschnitts entsprechen, das gemittelte Verhältnis von Flüssigkeits- zu Dampfstrom hinsichtlich der Flüssigkeit um nicht mehr als 15 %, bevorzugt um nicht mehr als 3 % überschritten werden. Diese niedrig gehaltene Flüssigkeitsmenge macht es möglich, dass der Kapillareffekt an den Drahtgeweben lokale Spitzenwerte der Flüssigkeitsberieselungsdichte ausschließt.

Bei Kolonnen mit unstrukturierten Packungen, insbesondere mit Füllkörpern, und bei Kolonnen mit strukturierten Packungen kann die gewünschte Charakteristik der Flüssigkeitsverteilung dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber den übrigen Querschnittsbereichen um bis zu 100 %, bevorzugt um 5 bis 15 %, verringert wird. Dies lässt sich zum Beispiel durch die gezielte Verteilung der Abtropfstellen der Flüssigkeitsverteiler oder deren Bohrungen mit einfachen Mitteln erreichen.

Das erfindungsgemäße Verfahren kann sowohl kontinuierlich als auch diskontinuierlich erfolgen. Bevorzugt erfolgt es kontinuierlich.

"Umsetzung eines Eduktstroms **S_{AE1}** umfassend ROH mit einem Eduktstrom **S_{AE2}** umfassend M_{A}OH im Gegenstrom " wird erfindungsgemäß insbesondere dadurch gewährleistet, dass der Zugabepunkt mindestens eines Teils des Eduktstroms **S_{AE1}** umfassend ROH in Schritt (a1) an der Reaktionskolonne **RR_{A}** unterhalb des Zugabepunktes des Eduktstroms **S_{AE2}** umfassend M_{A}OH liegt.

Die Reaktionskolonne **RR_{A}** umfasst vorzugsweise mindestens 2, insbesondere 15 bis 40 theoretische Böden zwischen dem Zugabepunkt des Eduktstroms **S_{AE1}** und dem Zugabepunkt des Eduktstroms **S_{AE2}.**

Die Reaktionskolonne **RR_{A}** wird bevorzugt als reine Abtriebskolonne betrieben. Insbesondere wird demnach im unteren Bereich der Reaktionskolonne **RR_{A}** dampfförmig der Eduktstrom **S_{AE1}** umfassend ROH zugeführt. Schritt (a1) des erfindungsgemäßen Verfahrens umfasst auch den Fall, dass ein Teil des Eduktstroms **S_{AE1}** umfassend ROH zwar unterhalb des Zugabepunktes des Eduktstroms **S_{AE2}** umfassend Alkalilauge M_{A}OH, aber dennoch am oberen Ende oder im Bereich des oberen Endes der Reaktionskolonne **RR_{A}** dampfförmig zugegeben wird. Dadurch können die Abmessungen im unteren Bereich der Reaktionskolonne **RR_{A}** verringert werden. Wenn ein Teil des Eduktstroms **S_{AE1}** umfassend ROH, insbesondere Methanol, am oberen Ende oder im Bereich des oberen Endes der Reaktionskolonne **RR_{A}** insbesondere dampfförmig zugegeben wird, so wird nur eine Teilmenge von 10 bis 70 Gew.-%, bevorzugt von 30 bis 50 Gew.-% (jeweils bezogen auf die Gesamtmenge des in Schritt (a1) eingesetzten Alkohols ROH) am unteren Ende der Reaktionskolonne **RR_{A}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{AE2}** umfassend M_{A}OH dampfförmig zugegeben.

In der Reaktionskolonne **RR_{A}** setzt sich dann der Eduktstrom **S_{AE1}** umfassend ROH mit dem Eduktstrom **S_{AE2}** umfassend M_{A}OH gemäß der vorstehend beschriebenen Reaktion **<1>** zu M_{A}OR und H₂O um, wobei, da es sich um eine Gleichgewichtsreaktion handelt, diese Produkte in Mischung mit den Edukten ROH und M_{A}OH vorliegen. Demnach wird in Schritt (a1) des erfindungsgemäßen Verfahrens in der Reaktionskolonne RR_{A} ein Rohprodukt **RP_{A}** erhalten, welches neben den Produkten M_{A}OR und Wasser auch noch ROH und M_{A}OH umfasst.

Am unteren Ende von **RR_{A}** erhält und entnimmt man dann den Sumpfproduktstrom **S_{AP}** umfassend ROH und M_{A}OR.

Am oberen Ende von **RR_{A},** bevorzugt am Kolonnenkopf von **RR_{A},** entnimmt man noch Wasser enthaltenden Alkoholstrom, vorstehend als "Brüdenstrom S_{AB} umfassend Wasser und ROH" bezeichnet.

Dieser Brüdenstrom **S_{AB}** umfassend Wasser und ROH wird dem Schritt (b) des erfindungsgemäßen Verfahrens zugeführt. Ein Teil des bei der Destillation in Schritt (b) gewonnenen Alkohols kann als Teil mindestens eines der Ströme **S_{B2}, S_{B3}, S_{B4}** der Reaktionskolonne **RR_{A}** als Eduktstrom **S_{AE1}** zugeführt werden.

Die Menge des vom Eduktstrom **S_{AE1}** umfassten Alkohols ROH wird vorzugsweise so gewählt, dass dieser gleichzeitig als Lösungsmittel für das im Sumpfproduktstrom **S_{AP}** erhaltene Alkalialkoholat M_{A}OR dient. Vorzugsweise wird die Menge des Alkohols ROH im Eduktstrom **S_{AE1}** so gewählt, dass im Sumpf der Reaktionskolonne die gewünschte Konzentration der Alkalialkoholat-Lösung vorliegt, die als Sumpfproduktstrom **S_{AP}** umfassend ROH und M_{A}OR entnommen wird.

In einer bevorzugten Ausführungsform des erfindungsgemäßen Verfahrens, und insbesondere in den Fällen, in denen **S_{AE2}** neben M_{A}OH auch Wasser umfasst, beträgt das Verhältnis des Gesamtgewichts (Massen; Einheit: kg) an in Schritt (a1) als Eduktstrom **S_{AE1}** eingesetztem Alkohol ROH zum Gesamtgewicht (Massen; Einheit: kg) an in Schritt (a1) als Eduktstrom **S_{AE2}** eingesetzten M_{A}OH 4 : 1 bis 50 : 1, bevorzugter 9 : 1 bis 48 : 1, noch bevorzugter 13 : 1 bis 35 : 1, noch mehr bevorzugter 22 : 1 bis 30 : 1.

Die Reaktionskolonne **RR_{A}** wird mit oder ohne, vorzugsweise ohne Rücklauf betrieben.

"Ohne Rücklauf" bedeutet, dass der am oberen Ende von **RR_{A}** entnommene Brüdenstrom **S_{AB}** umfassend Wasser und ROH der Rektifikationskolonne **RD_{A}** gemäß Schritt (b) vollständig zugeführt wird. Der Brüdenstrom **S_{AB}** umfassend Wasser und ROH wird der Rektifikationskolonne **RD_{A}** dabei vorzugsweise dampfförmig zugeführt.

"Mit Rücklauf" bedeutet, dass der am oberen Ende der jeweiligen Kolonne, in Schritt (a1) der Reaktionskolonne **RR_{A},** entnommene Brüdenstrom **S_{AB}** umfassend Wasser und ROH nicht vollständig abgeführt wird, in Schritt (b) also nicht vollständig der Rektifikationskolonne **RD_{A}** zugeführt wird, sondern mindestens teilweise, bevorzugt teilweise, wieder als Rücklauf der jeweiligen Kolonne, in Schritt (a1) der Reaktionskolonne **RR_{A},** zugeführt wird. In den Fällen, in denen ein solcher Rücklauf eingestellt wird, beträgt das Rücklaufverhältnis dabei bevorzugt 0.05 bis 0.99, bevorzugter 0.1 bis 0.9, noch bevorzugter 0.11 bis 0.34, besonderes bevorzugt 0.14 bis 0.27 und ganz besonders bevorzugt 0.17 bis 0.24. Ein Rücklauf kann dadurch eingestellt werden, dass am Kopf der jeweiligen Kolonne, in Schritt (a1) der Reaktionskolonne **RR_{A},** ein Kondensator **K_{RRA}** angebracht wird, in welchem der Brüdenstrom **S_{AB}** mindestens teilweise kondensiert wird und der jeweiligen Kolonne, in Schritt (a1) der Reaktionskolonne **RR_{A},** wieder zugeführt wird. Unter einem Rücklaufverhältnis wird allgemein und im Sinne dieser Erfindung das Verhältnis des Massenstroms (kg/h) verstanden, der der jeweiligen Kolonne in flüssiger Form zurückgeführt wird (Rücklauf) und des Massenstroms (kg/h), der in flüssiger Form (Destillat) oder gasförmiger Form (Brüden) von der jeweiligen Kolonne abgeführt wird.

In der Ausführungsform, in der an der Reaktionskolonne **RR_{A}** ein Rücklauf eingestellt wird, kann der in Schritt (a1) als Eduktstrom **S_{AE2}** eingesetzte M_{A}OH auch mindestens teilweise mit dem Rücklaufstrom vermischt werden und die resultierende Mischung so dem Schritt (a1) zugeführt werden.

Der Schritt (a1) des erfindungsgemäßen Verfahrens wird insbesondere bei einer Temperatur im Bereich von 45 °C bis 150 °C, bevorzugt 47 °C bis 120 °C, bevorzugter 60 °C bis 110 °C, und bei einem Druck von 0.5 bar bis 40 bar, bevorzugt im Bereich von 0.75 bar bis 5 bar, bevorzugter im Bereich von 1 bar bis 2 bar, bevorzugter im Bereich von 1 bar bis 1.5 bar, noch bevorzugter bei Umgebungsdruck (1 bar), durchgeführt.

Die Reaktionskolonne **RR_{A}** umfasst in einer bevorzugten Ausführungsform mindestens einen Verdampfer, der insbesondere aus Zwischenverdampfern **V_{ZA}** und Sumpfverdampfern **V_{SA}** ausgewählt ist. Die Reaktionskolonne **RR_{A}** umfasst besonders bevorzugt mindestens einen Sumpfverdampfer **V_{SA}.**

Als "Zwischenverdampfer" **V_{ZA}** und **V_{ZB}** werden erfindungsgemäß Verdampfer bezeichnet, die sich oberhalb des Sumpfs der jeweiligen Kolonne, insbesondere oberhalb des Sumpfs der Reaktionskolonne **RR_{A}** bzw. **RR_{B},** befinden. In ihnen wird insbesondere Rohprodukt **RP_{A}** bzw. **RP_{B}** verdampft.

Als "Sumpfverdampfer" **V_{SA}** und **V_{SB}** werden erfindungsgemäß Verdampfer bezeichnet, die den Sumpf der jeweiligen Kolonne, insbesondere den Sumpf der Reaktionskolonne **RR_{A}** bzw. **RR_{B},** beheizen. In ihnen wird Sumpfproduktstrom **S_{AP}** bzw. **S_{BP}** verdampft.

Ein Verdampfer ist üblicherweise außerhalb der jeweiligen Reaktionskolonne oder Rektifikationskolonne angeordnet. Über einen Abzug aus der Kolonne wird das im Verdampfer zu verdampfende Gemisch abgezogen und dem mindestens einen Verdampfer zugeführt. Im Falle der Reaktionskolonne **RR_{A}** bzw. **RR_{B}** wird bei der Zwischenverdampfung des Rohprodukts **RP_{A}** bzw. **RP_{B},** dieses abgezogen und dem mindestens einen Zwischenverdampfer **V_{ZA}** bzw. **V_{ZB}** zugeführt. Über einen Zulauf wird das verdampfte Gemisch gegebenenfalls mit einem Restanteil Flüssigkeit wieder in die jeweilige Kolonne zurückgeführt. Wenn der Verdampfer ein Zwischenverdampfer ist, es sich also insbesondere um einen Zwischenverdampfer **V_{ZA}** bzw. **V_{ZB}** handelt, ist der Abzug, über den das jeweilige Gemisch abgezogen und dem Verdampfer zugeführt wird, ein Seitenabzug und der Zulauf, über den das verdampfte jeweilige Gemisch der Kolonne wieder zugeführt wird, ein Seitenzulauf. Wenn der Verdampfer ein Sumpfverdampfer ist, also den Kolonnensumpf beheizt, es sich also insbesondere um einen Sumpfverdampfer **V_{SA}** bzw. **V_{SB}** handelt, so wird zumindest ein Teil des Sumpfabzugsstroms, insbesondere **S_{AP}** bzw. **S_{BP},** dem Sumpfverdampfer **V_{S}** zugeführt, verdampft und im Bereich des Sumpfs wieder in die jeweilige Kolonne zurückgeführt. Alternativ ist es jedoch auch möglich, zum Beispiel auf einem geeigneten Boden bei Einsatz eines Zwischenverdampfers oder im Sumpf der jeweiligen Kolonne Rohre auszubilden, die von dem jeweiligen verdichteten Brüdenstrom **S_{B3}** bzw. einem Wärmemedium **W,** durchströmt werden. In diesem Fall erfolgt die Verdampfung auf dem Boden bzw. im Sumpf der Kolonne. Bevorzugt ist es jedoch, den Verdampfer außerhalb der jeweiligen Kolonne anzuordnen.

Geeignete Verdampfer, die als Zwischenverdampfer und Sumpfverdampfer eingesetzt werden können, sind zum Beispiel Naturumlaufverdampfer, Zwangsumlaufverdampfer, Zwangsumlaufverdampfer mit Entspannung, Dampfkessel, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann der verdichtete Brüdenstrom **S_{B3}** bzw. das Wärmemedium **W,** entweder durch die Rohre strömen und das zu verdampfende Gemisch die Rohre umströmen oder aber der verdichtete Brüdenstrom **S_{B3}** bzw. das Wärmemedium **W,** umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne eignet, eingesetzt werden.

Wenn der Verdampfer, der mit dem verdichteten Brüdenstrom **S_{B3}** bzw. das Wärmemedium W, als Heizdampf betrieben wird, ein Zwischenverdampfer ist, ist es bevorzugt, wenn der Zwischenverdampfer im Abtriebsteil der Reaktionskolonne **RR_{A}** im Bereich der Zulaufstelle des Eduktstroms **S_{AE1}** bzw. im Falle der Reaktionskolonne **RR_{B}** im Bereich der Zulaufstelle des Eduktstroms **S_{BE1}** angeordnet ist. Dadurch kann ein überwiegender Teil der Heizenergie durch den Zwischenverdampfer eingebracht werden. So ist es zum Beispiel möglich, über 80% der Energie über den Zwischenverdampfer einzubringen. Erfindungsgemäß wird der Zwischenverdampfer vorzugsweise so angeordnet und/oder gestaltet, dass mit diesem mehr als 50%, insbesondere mehr als 75% der für die Destillation benötigten Gesamtenergie eingebracht werden.

Bei Einsatz eines Zwischenverdampfers ist es insbesondere vorteilhaft, wenn der Zwischenverdampfer so angeordnet ist, dass die Reaktionskolonne **RR_{A}** bzw. **RR_{B}** unterhalb des Zwischenverdampfers 1 bis 50 theoretische Böden aufweist und oberhalb des Zwischenverdampfers 1 bis 200 theoretische Böden. Insbesondere ist es bevorzugt, wenn die Reaktionskolonne **RR_{A}** bzw. **RR_{B}** unterhalb des Zwischenverdampfers 2 bis 10 theoretische Böden aufweist und oberhalb des Zwischenverdampfers 20 bis 50 theoretische Böden.

Der Seitenabzugsstrom, über den das Rohprodukt **RP_{A}** bzw. **RP_{B}** dem Zwischenverdampfer **V_{ZA}** bzw. **V_{ZB}** zugeführt wird, und der Seitenzulauf, über den das verdampfte Rohprodukt **RP_{A}** bzw. **RP_{B}** aus dem Zwischenverdampfer **V_{ZA}** bzw. **V_{ZB}** wieder der jeweiligen Reaktionskolonne **RR_{A}** bzw. **RR_{B}** zugeführt wird, können zwischen den gleichen Böden der Reaktionskolonne positioniert sein. Es ist jedoch auch möglich, dass Seitenabzug und Seitenzulauf auf unterschiedlicher Höhe liegen.

In einer bevorzugten Ausführungsform ist bei Einsatz eines Zwischenverdampfers **RR_{A}** bzw. **RR_{B}** der Durchmesser der Reaktionskolonne **RR_{A}** bzw. **RR_{B}** oberhalb des Zwischenverdampfers **RR_{A}** bzw. **RR_{B}** größer als der Durchmesser der Reaktionskolonne **RR_{A}** bzw. **RR_{B}** unterhalb des Zwischenverdampfers **RR_{A}** bzw. **RR_{B}.** Dies hat den Vorteil, dass Investitionskosten gespart werden können.

In einem solchen Zwischenverdampfer **V_{ZA}** kann in der Reaktionskolonne **RR_{A}** befindliches, flüssiges Rohprodukt **RP_{A}** umfassend M_{A}OR, Wasser, ROH, M_{A}OH in den gasförmigen Zustand überführt werden, und so die Effizienz der Umsetzung gemäß Schritt (a1) des erfindungsgemäßen Verfahrens verbessert werden.

Durch die Anordnung von einem oder mehreren Zwischenverdampfern **V_{ZA}** im oberen Bereich der Reaktionskolonne **RR_{A}** können die Abmessungen im unteren Bereich der Reaktionskolonne **RR_{A}** verringert werden. Bei der Ausführungsform mit mindestens einem, bevorzugt mehreren Zwischenverdampfern **V_{ZA}** ist es auch möglich, Teilströme des ROH in flüssiger Form im oberen Bereich der Reaktionskolonne **RR_{A}** zuzuführen.

Sumpfverdampfer sind erfindungsgemäß am Sumpf der Reaktionskolonne **RR_{A}** bzw. **RR_{B}** angeordnet und werden dann als **"V_{SA}"** bzw. **"V_{SB}"** bezeichnet. In einem solchen Sumpfverdampfer kann in der Reaktionskolonne **RR_{A}** bzw. **RR_{B}** befindlicher Sumpfproduktstrom **S_{AP}** bzw. **S_{BP}** geleitet und ROH mindestens teilweise daraus entfernt werden, wodurch ein Sumpfproduktstrom **S_{AP*}** mit einem gegenüber **S_{AP}** erhöhten Massenanteil an M_{A}OR erhalten wird bzw. wodurch ein Sumpfproduktstrom **S_{BP*}** mit einem gegenüber **S_{BP}** erhöhten Massenanteil an M_{B}OR erhalten wird. In Schritt (a1) des erfindungsgemäßen Verfahrens wird am unteren Ende der Reaktionskolonne **RR_{A}** ein Sumpfproduktstrom **S_{AP}** umfassend ROH und M_{A}OR entnommen.

Es ist bevorzugt, dass die Reaktionskolonne **RR_{A}** mindestens einen Sumpfverdampfer **V_{SA}** aufweist, über den Sumpfproduktstrom **S_{AP}** dann mindestens teilweise geleitet wird und ROH aus diesem mindestens teilweise entfernt wird, wodurch ein Sumpfproduktstrom **S_{AP*}** mit einem gegenüber **S_{AP}** erhöhten Massenanteil an M_{A}OR erhalten wird. In dieser weiteren bevorzugten Ausführungsform ist es noch vorteilhafter, wenn in Schritt (d) **S_{B3}** oder **W₁** mindestens teilweise über einen Sumpfverdampfer **V_{SA}** geleitet wird und die Energie von **S_{B3}** bzw. **W,** auf den Sumpfproduktstrom **S_{AP}** übertragen wird, insbesondere in dem **S_{B3}** bzw. **W,** zur Beheizung des Verdampfers **V_{A1}** genutzt wird. **W₁** ist ein von **RP_{A}, S_{AE1}, S_{AE2}, S_{AP}, S_{AB}, RP_{B}, S_{BE1}, S_{BE2}, S_{BP}** oder **S_{BB}** verschiedener Wärmeträger, der **S_{B3}** kontaktiert hat, so dass Energie von **S_{B3}** auf den mindestens einen Wärmeträger **W,** übergeht (siehe Abschnitt 4.5).

Der Massenanteil an M_{A}OR des Sumpfproduktstroms **S_{AP*}** ist dabei insbesondere gegenüber dem Massenanteil an M_{A}OR des Sumpfproduktstroms **S_{AP}** um mindestens 1 % erhöht, bevorzugt um ≥ 2 %, bevorzugter um ≥ 5 %, noch bevorzugter um ≥ 10 %, noch mehr bevorzugter um ≥ 20 %, noch mehr bevorzugter um ≥ 30 %, noch mehr bevorzugter um ≥ 40 %, noch mehr bevorzugter um ≥ 50 %, noch mehr bevorzugter um ≥ 100 %, noch mehr bevorzugter um ≥ 150 %.

Bevorzugt weist **S_{AP}** oder, falls mindestens einen Sumpfverdampfer **V_{SA}** eingesetzt wird, über den der Sumpfproduktstrom **S_{AP}** mindestens teilweise geleitet wird und ROH aus diesem mindestens teilweise entfernt wird, **S_{AP*},** einen Massenanteil von M_{A}OR in ROH im Bereich 1 bis 50 Gew.-%, bevorzugt 5 bis 32 Gew.-%, bevorzugter 15 bis 32 Gew.-%, am bevorzugtesten 30 bis 32 Gew.-% auf, jeweils bezogen auf die Gesamtmasse von **S_{AP}.**

Der Massenanteil an Restwasser in **S_{AP}** bzw. **S_{AP*}** liegt dabei bevorzugt bei < 1 Gew.-%, bevorzugt < 0.1 Gew.-%, bevorzugter < 0.01 Gew.-%, bezogen auf die Gesamtmasse von **S_{AP}.**

Der Massenanteil an Edukt M_{A}OH in **S_{AP}** bzw. **S_{AP*}** liegt dabei bevorzugt bei < 1 Gew.-%, bevorzugt < 0.1 Gew.-%, bevorzugter < 0.01 Gew.-%, bezogen auf die Gesamtmasse von **S_{AP}.**

### 4.2 Schritt (a2) des erfindungsgemäßen Verfahrens (optional)

Schritt (a2) wird erfindungsgemäß optional durchgeführt. Im optionalen Schritt (a2), der gleichzeitig mit und räumlich getrennt von Schritt (a1) des erfindungsgemäßen Verfahrens abläuft, wird ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohprodukt **RP_{B}** umfassend M_{B}OR, Wasser, ROH, M_{B}OH umgesetzt.

In Schritt (a2) des erfindungsgemäßen Verfahrens wird am unteren Ende von **RR_{B}** ein Sumpfproduktstrom **S_{BP}** umfassend ROH und M_{B}OR entnommen. Am oberen Ende von **RR_{B}** wird ein Brüdenstrom **S_{BB}** umfassend Wasser und ROH entnommen.

M_{B} ist ausgewählt aus Natrium, Kalium, und bevorzugt Kalium.

Der Eduktstrom **S_{BE1}** umfasst ROH. In einer bevorzugten Ausführungsform liegt der Massenanteil von ROH in **S_{BE1}** bei ≥ 95 Gew.-%, noch bevorzugter bei ≥ 99 Gew.-%, wobei **S_{BE1}** ansonsten insbesondere Wasser aufweist.

Der in Schritt (a2) des erfindungsgemäßen Verfahrens als Eduktstrom **S_{BE1}** eingesetzte Alkohol ROH kann auch handelsüblicher Alkohol mit einem Alkoholmassenanteil von mehr als 99.8 Gew.-% und einem Massenanteil an Wasser von bis zu 0.2 Gew.-% sein.

Der Eduktstrom **S_{BE1}** wird bevorzugt dampfförmig zugegeben.

Der Eduktstrom **S_{BE2}** umfasst M_{B}OH. In einer bevorzugten Ausführungsform umfasst **S_{BE2}** neben M_{B}OH mindestens eine weitere Verbindung ausgewählt aus Wasser, ROH. Noch bevorzugter umfasst **S_{BE2}** neben M_{B}OH Wasser, dann handelt es sich bei **S_{BE2}** um eine wässrige Lösung von M_{B}OH.

Wenn der Eduktstrom **S_{BE2}** M_{B}OH und Wasser umfasst, liegt der Massenanteil von M_{B}OH, bezogen auf das Gesamtgewicht der wässrigen Lösung, welche **S_{BE2}** bildet, insbesondere im Bereich von 10 bis 55 Gew.-%, bevorzugt von 15 bis 54 Gew.-%, bevorzugter von 30 bis 53 Gew.-% und besonders bevorzugt von 45 bis 52 Gew.-%. Am bevorzugtesten liegt der Anteil von M_{B}OH in der wässrigen Lösung dann bei 50 Gew.-%.

Wenn der Eduktstrom **S_{BE2}** M_{B}OH und ROH umfasst, liegt der Massenanteil von M_{B}OH in ROH, bezogen auf das Gesamtgewicht der Lösung, welche **S_{BE2}** bildet, insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-%.

In dem besonderen Fall, in dem der Eduktstrom **S_{BE2}** neben M_{B}OH sowohl Wasser als auch ROH umfasst, ist es besonders bevorzugt, dass der Massenanteil von M_{B}OH in ROH und Wasser, bezogen auf das Gesamtgewicht der Lösung, welche **S_{BE2}** bildet, insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-% liegt.

Schritt (a2) des erfindungsgemäßen Verfahrens wird in einer Reaktivrektifikationskolonne (oder "Reaktionskolonne") **RR_{B}** durchgeführt. Bevorzugte Ausführungsformen der Reaktionskolonne **RR_{B}** sind im Abschnitt 4.1 beschrieben.

"Umsetzung eines Eduktstroms **S_{BE1}** umfassend ROH mit einem Eduktstrom **S_{BE2}** umfassend M_{B}OH im Gegenstrom " wird erfindungsgemäß insbesondere dadurch gewährleistet, dass der Zugabepunkt mindestens eines Teils des Eduktstroms **S_{BE1}** umfassend ROH in Schritt (a2) an der Reaktionskolonne **RR_{B}** unterhalb des Zugabepunktes des Eduktstroms **S_{BE2}** umfassend M_{B}OH liegt.

Die Reaktionskolonne **RR_{B}** umfasst vorzugsweise mindestens 2, insbesondere 15 bis 40 theoretische Böden zwischen dem Zugabepunkt des Eduktstroms **S_{BE1}** und dem Zugabepunkt des Eduktstroms **S_{BE2}.**

Die Reaktionskolonne **RR_{B}** wird bevorzugt als reine Abtriebskolonne betrieben. Insbesondere wird demnach im unteren Bereich der Reaktionskolonne **RR_{B}** dampfförmig der Eduktstrom **S_{BE1}** umfassend ROH zugeführt. Schritt (a2) des erfindungsgemäßen Verfahrens umfasst auch den Fall, dass ein Teil des Eduktstroms **S_{BE1}** umfassend ROH zwar unterhalb des Zugabepunktes des Eduktstroms **S_{BE2}** umfassend Alkalilauge M_{B}OH, aber dennoch am oberen Ende oder im Bereich des oberen Endes der Reaktionskolonne **RR_{B}** dampfförmig zugegeben wird. Dadurch können die Abmessungen im unteren Bereich der Reaktionskolonne **RR_{B}** verringert werden. Wenn ein Teil des Eduktstroms **S_{BE1}** umfassend ROH, insbesondere Methanol, am oberen Ende oder im Bereich des oberen Endes der Reaktionskolonne **RR_{B}** insbesondere dampfförmig zugegeben wird, so wird nur eine Teilmenge von 10 bis 70 Gew.-%, bevorzugt von 30 bis 50 Gew.-% (jeweils bezogen auf die Gesamtmenge des in Schritt (a2) eingesetzten Alkohols ROH) am unteren Ende der Reaktionskolonne **RR_{B}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{BE2}** umfassend M_{B}OH dampfförmig zugegeben.

In der Reaktionskolonne **RR_{B}** setzt sich dann der Eduktstrom **S_{BE1}** umfassend ROH mit dem Eduktstrom **S_{BE2}** umfassend M_{B}OH gemäß der vorstehend beschriebenen Reaktion **<1>** zu M_{B}OR und H₂O um, wobei, da es sich um eine Gleichgewichtsreaktion handelt, diese Produkte in Mischung mit den Edukten ROH und M_{B}OH vorliegen. Demnach wird in Schritt (a2) des erfindungsgemäßen Verfahrens in der Reaktionskolonne **RR_{B}** ein Rohprodukt **RP_{B}** erhalten, welches neben den Produkten M_{B}OR und Wasser auch noch ROH und M_{B}OH umfasst.

Am unteren Ende von **RR_{B}** erhält und entnimmt man dann den Sumpfproduktstrom **S_{BP}** umfassend ROH und M_{B}OR.

Am oberen Ende von **RR_{B},** bevorzugt am Kolonnenkopf von **RR_{B},** entnimmt man noch Wasser enthaltenden Alkoholstrom, vorstehend als "Brüdenstrom S_{BB} umfassend Wasser und ROH" bezeichnet.

Dieser Brüdenstrom **S_{BB}** umfassend Wasser und ROH wird dem Schritt (b) des erfindungsgemäßen Verfahrens zugeführt. Dabei wird er, bevor er dem Schritt (b) des erfindungsgemäßen Verfahrens zugeführt wird, mit **S_{AB}** vermischt oder nicht, also getrennt von **S_{AB}** dem Schritt (b) des erfindungsgemäßen Verfahrenszugeführt. Bevorzugt wird Brüdenstrom **S_{BB}** mit **S_{AB}** vermischt und dann die Mischung dem Schritt (b) des erfindungsgemäßen Verfahrens zugeführt. Ein Teil des bei der Destillation in Schritt (b) gewonnenen Alkohols kann als Teil mindestens eines der Ströme **S_{B2}, S_{B3}, S_{B4}** der Reaktionskolonne **RR_{B}** als Eduktstrom **S_{BE1}** zugeführt werden.

Die Menge des vom Eduktstrom S_{BE1} umfassten Alkohols ROH wird vorzugsweise so gewählt, dass dieser gleichzeitig als Lösungsmittel für das im Sumpfproduktstrom **S_{BP}** erhaltene Alkalialkoholat M_{B}OR dient. Vorzugsweise wird die Menge des Alkohols ROH im Eduktstrom **S_{BE1}** so gewählt, dass im Sumpf der Reaktionskolonne die gewünschte Konzentration der Alkalialkoholat-Lösung vorliegt, die als Sumpfproduktstrom **S_{BP}** umfassend ROH und M_{B}OR entnommen wird.

In einer bevorzugten Ausführungsform des erfindungsgemäßen Verfahrens, und insbesondere in den Fällen, in denen **S_{BE2}** neben M_{B}OH auch Wasser umfasst, beträgt das Verhältnis des Gesamtgewichts (Massen; Einheit: kg) an in Schritt (a2) als Eduktstrom **S_{BE1}** eingesetztem Alkohol ROH zum Gesamtgewicht (Massen; Einheit: kg) an in Schritt (a2) als Eduktstrom **S_{BE2}** eingesetzten M_{B}OH 4 : 1 bis 50 : 1, bevorzugter 9 : 1 bis 48 : 1, noch bevorzugter 13 : 1 bis 35 : 1, noch mehr bevorzugter 22 : 1 bis 30 : 1.

Die Reaktionskolonne **RR_{B}** wird mit oder ohne, vorzugsweise ohne Rücklauf betrieben.

"Ohne Rücklauf" bedeutet, dass der am oberen Ende von **RR_{B}** entnommene Brüdenstrom **S_{BB}** umfassend Wasser und ROH der Rektifikationskolonne **RD_{A}** gemäß Schritt (b) vollständig zugeführt wird. Der Brüdenstrom **S_{BB}** umfassend Wasser und ROH wird der Rektifikationskolonne **RD_{A}** dabei vorzugsweise dampfförmig zugeführt.

"Mit Rücklauf" bedeutet, dass der am oberen Ende der jeweiligen Kolonne, in Schritt (a2) der Reaktionskolonne **RR_{B},** entnommene Brüdenstrom **S_{BB}** umfassend Wasser und ROH nicht vollständig abgeführt wird, in Schritt (b) also nicht vollständig der Rektifikationskolonne **RD_{A}** zugeführt wird, sondern mindestens teilweise, bevorzugt teilweise, wieder als Rücklauf der jeweiligen Kolonne, in Schritt (a2) der Reaktionskolonne **RR_{B},** zugeführt wird. In den Fällen, in denen ein solcher Rücklauf eingestellt wird, beträgt das Rücklaufverhältnis dabei bevorzugt 0.05 bis 0.99, bevorzugter 0.1 bis 0.9, noch bevorzugter 0.11 bis 0.34, besonderes bevorzugt 0.14 bis 0.27 und ganz besonders bevorzugt 0.17 bis 0.24. Ein Rücklauf kann dadurch eingestellt werden, dass am Kopf der jeweiligen Kolonne, in Schritt (a2) der Reaktionskolonne **RR_{B},** ein Kondensator **K_{RRB}** angebracht wird, in welchem der Brüdenstrom **S_{BB}** mindestens teilweise kondensiert wird und der jeweiligen Kolonne, in Schritt (a2) der Reaktionskolonne **RR_{B},** wieder zugeführt wird.

In der Ausführungsform, in der an der Reaktionskolonne **RR_{B}** ein Rücklauf eingestellt wird, kann der in Schritt (a2) als Eduktstrom **S_{BE2}** eingesetzte M_{B}OH auch mindestens teilweise mit dem Rücklaufstrom vermischt werden und die resultierende Mischung so dem Schritt (a2) zugeführt werden.

Der Schritt (a2) des erfindungsgemäßen Verfahrens wird insbesondere bei einer Temperatur im Bereich von 45 °C bis 150 °C, bevorzugt 47 °C bis 120 °C, bevorzugter 60 °C bis 110 °C, und bei einem Druck von 0.5 bar bis 40 bar, bevorzugt im Bereich von 0.75 bar bis 5 bar, bevorzugter im Bereich von 1 bar bis 2 bar, bevorzugter im Bereich von 1 bar bis 1.5 bar, noch bevorzugter bei Umgebungsdruck (1 bar), durchgeführt.

Die Reaktionskolonne **RR_{B}** umfasst in einer bevorzugten Ausführungsform mindestens einen Verdampfer, der insbesondere aus Zwischenverdampfern **V_{ZB}** und Sumpfverdampfern **V_{SB}** ausgewählt ist. Die Reaktionskolonne **RR_{B}** umfasst besonders bevorzugt mindestens einen Sumpfverdampfer **V_{SB}.**

In einem solchen Zwischenverdampfer **V_{ZB}** kann in der Reaktionskolonne **RR_{B}** befindliches, flüssiges Rohprodukt **RP_{B}** umfassend M_{B}OR, Wasser, ROH, M_{B}OH in den gasförmigen Zustand überführt werden, und so die Effizienz der Umsetzung gemäß Schritt (a2) des erfindungsgemäßen Verfahrens verbessert werden.

Durch die Anordnung von einem oder mehreren Zwischenverdampfern **V_{ZB}** im oberen Bereich der Reaktionskolonne **RR_{B}** können die Abmessungen im unteren Bereich der Reaktionskolonne **RR_{B}** verringert werden. Bei der Ausführungsform mit mindestens einem, bevorzugt mehreren Zwischenverdampfern **V_{ZB}** ist es auch möglich, Teilströme des ROH in flüssiger Form im oberen Bereich der Reaktionskolonne **RR_{B}** zuzuführen.

In Schritt (a2) des erfindungsgemäßen Verfahrens wird am unteren Ende der Reaktionskolonne **RR_{B}** ein Sumpfproduktstrom **S_{BP}** umfassend ROH und M_{B}OR entnommen.

Es ist bevorzugt, dass die Reaktionskolonne **RR_{B}** mindestens einen Sumpfverdampfer **V_{SB}** aufweist, über den Sumpfproduktstrom **S_{BP}** dann mindestens teilweise geleitet wird und ROH aus diesem mindestens teilweise entfernt wird, wodurch ein Sumpfproduktstrom **S_{BP*}** mit einem gegenüber **S_{BP}** erhöhten Massenanteil an M_{B}OR erhalten wird. In dieser weiteren bevorzugten Ausführungsform ist es noch vorteilhafter, wenn in Schritt (d) **S_{B3}** oder **W₁** mindestens teilweise über einen Sumpfverdampfer **V_{SB}** geleitet wird und die Energie von **S_{B3}** bzw. **W,** auf den Sumpfproduktstrom **S_{BP}** übertragen wird, insbesondere in dem **S_{B3}** bzw. **W,** zur Beheizung des Verdampfers **V_{B1}** genutzt wird.

Der Massenanteil an M_{B}OR des Sumpfproduktstroms **S_{BP*}** ist dabei insbesondere gegenüber dem Massenanteil an M_{B}OR des Sumpfproduktstroms **S_{BP}** um mindestens 1 % erhöht, bevorzugt um ≥ 2 %, bevorzugter um ≥ 5 %, noch bevorzugter um ≥ 10 %, noch mehr bevorzugter um ≥ 20 %, noch mehr bevorzugter um ≥ 30 %, noch mehr bevorzugter um ≥ 40 %, noch mehr bevorzugter um ≥ 50 %, noch mehr bevorzugter um ≥ 100 %, noch mehr bevorzugter um ≥ 150 %.

Bevorzugt weist **S_{BP}** oder, falls mindestens einen Sumpfverdampfer **V_{SB}** eingesetzt wird, über den der Sumpfproduktstrom **S_{BP}** mindestens teilweise geleitet wird und ROH aus diesem mindestens teilweise entfernt wird, **S_{BP*},** einen Massenanteil von M_{B}OR in ROH im Bereich 1 bis 50 Gew.-%, bevorzugt 5 bis 32 Gew.-%, bevorzugter 15 bis 32 Gew.-%, am bevorzugtesten 30 bis 32 Gew.-% auf, jeweils bezogen auf die Gesamtmasse von **S_{BP}.**

Der Massenanteil an Restwasser in **S_{BP}** bzw. **S_{BP*}** liegt dabei bevorzugt bei < 1 Gew.-%, bevorzugt < 0.1 Gew.-%, bevorzugter < 0.01 Gew.-%, bezogen auf die Gesamtmasse von **S_{BP}.**

Der Massenanteil an Edukt M_{B}OH in **S_{BP}** bzw. **S_{BP*}** liegt dabei bevorzugt bei < 1 Gew.-%, bevorzugt < 0.1 Gew.-%, bevorzugter < 0.01 Gew.-%, bezogen auf die Gesamtmasse von **S_{BP}.**

In den Ausführungsformen des vorliegenden Verfahrens, in denen auch Schritt (a2) ausgeführt wird, wird bevorzugt der Sumpfproduktstrom **S_{AP}** mindestens teilweise über einen Sumpfverdampfer V_{SA} geleitet und ROH aus **S_{AP}** mindestens teilweise entfernt, wodurch ein Sumpfproduktstrom **S_{AP*}** mit einem gegenüber **S_{AP}** erhöhten Massenanteil an M_{A}OR erhalten wird und/oder, bevorzugt und, der Sumpfproduktstrom **S_{BP}** mindestens teilweise über einen Sumpfverdampfer **V_{SB}** geleitet und ROH aus **S_{BP}** mindestens teilweise entfernt, wodurch ein Sumpfproduktstrom **S_{BP*}** mit einem gegenüber **S_{BP}** erhöhten Massenanteil an M_{B}OR erhalten wird. In dieser weiteren bevorzugten Ausführungsform ist es noch vorteilhafter, wenn in Schritt (d) die Energie von **S_{B3}** auf den Sumpfproduktstrom **S_{AP}** und/oder, bevorzugt und, den Sumpfproduktstrom **S_{BP}** übertragen wird, insbesondere in dem **S_{B3}** zur Beheizung des Sumpfverdampfers **V_{SA}** und/oder, bevorzugt und, des Sumpfverdampfers **V_{SB}** genutzt wird. Diese Nutzung der Energie von **S_{B3}** kann direkt oder indirekt erfolgen.

Der Schritt (a2) des erfindungsgemäßen Verfahrens wird in den Ausführungsformen der vorliegenden Erfindung, in denen er durchgeführt wird, gleichzeitig mit und räumlich getrennt von Schritt (a1) durchgeführt. Die räumliche Trennung wird durch die Durchführung der Schritte (a1) und (a2) in den beiden Reaktionskolonnen **RR_{A}** und **RR_{B}** gewährleistet.

In einer vorteilhaften Ausgestaltung der Erfindung sind die Reaktionskolonnen **RR_{A}** und **RR_{B}** in einem Kolonnenmantel untergebracht, wobei die Kolonne mindestens teilweise durch mindestens eine Trennwand unterteilt ist. Eine solche mindestens eine Trennwand aufweisende Kolonne wird erfindungsgemäß als **"TRD"** bezeichnet. Solche Trennwandkolonnen sind dem Fachmann bekannt und beispielsweise beschrieben in US 2,295,256, EP 0 122 367 A2, EP 0 126 288 A2, WO 2010/097318 A1 sowie von I. Dejanović, Lj. Matijašević, Ž. Olujić, *Chemical Engineering and Processing* **2010,** *49*, 559-580. In den für das erfindungsgemäße Verfahren in Frage kommenden Trennwandkolonnen sind die Trennwände bevorzugt bis zum Boden durchgezogen und durchspannen insbesondere bevorzugt mindestens ein Viertel, bevorzugter mindestens ein Drittel, noch bevorzugter mindestens die Hälfte, noch bevorzugter mindesten zwei Drittel, noch mehr bevorzugter mindestens drei Viertel der Kolonne der Länge nach. Sie teilen die Kolonne in mindestens zwei Reaktionsräume, in denen räumlich getrennte Reaktionen stattfinden können. Die durch die mindestens eine Trennwand geschaffenen Reaktionsräume können gleich oder unterschiedlich groß sein.

In dem jeweils von der Trennwand getrennten Bereichen können in dieser Ausführungsform die Sumpfproduktströme **S_{AP}** und **S_{BP}** getrennt entnommen werden und bevorzugt über den für jeden durch die mindestens eine Reaktionswand gebildeten Reaktionsraum angebrachten Sumpfverdampfer **V_{SA}** bzw. **V_{SB}** geleitet werden , in dem ROH aus **S_{AP}** bzw. **S_{BP}** mindestens teilweise entfernt wird, wodurch **S_{AP*}** bzw. **S_{BP*}** erhalten werden.

### 4.3 Schritt (b) des erfindungsgemäßen Verfahrens

In Schritt (b) des erfindungsgemäßen Verfahrens wird der Brüdenstrom **S_{AB}** in eine Rektifikationskolonne **RD_{A}** geleitet, und in **RD_{A}** in einen ROH umfassenden Brüdenstrom **S_{B2}** mit einem Druck **p_{B2}** und einer Temperatur **T_{B2}** am Kopf von **RD_{A}** und einen Wasserstrom **S_{W}** am Sumpf von **RD_{A}** aufgetrennt.

In der optionalen Ausführung des erfindungsgemäßen Verfahrens, in der Schritt (a2) durchgeführt wird, wird der Brüdenstrom **S_{BB}** ebenfalls in eine Rektifikationskolonne **RD_{A}** geleitet, und in **RD_{A}** in einen ROH umfassenden Brüdenstrom **S_{B2}** mit einem Druck **p_{B2}** und einer Temperatur **T_{B2}** am Kopf von **RD_{A}** und einen Wasserstrom **S_{W}** am Sumpf von **RD_{A}** aufgetrennt. Der Brüdenstrom **S_{BB}** wird dabei vermischt mit **S_{AB}** oder getrennt von **S_{AB}** in eine Rektifikationskolonne **RD_{A}** geleitet. Der Brüdenstrom **S_{BB} wird** dabei bevorzugt vermischt mit **S_{AB}** und dann in eine Rektifikationskolonne **RD_{A}** geleitet.

Die Brüdenströme **S_{AB}** und gegebenenfalls **S_{BB}** können in einer Ausführungsform der vorliegenden Erfindung verdichtet werden, bevor sie in die Rektifikationskolonne **RD_{A}** geleitet werden. Dies kann über einen optionalen Verdichter **VD_{AB1}** geschehen.

Es versteht sich von selbst, dass auch in den Ausführungsformen, in denen **S_{BB}** getrennt von **S_{AB}** in eine Rektifikationskolonne **RD_{A}** geleitet wird, sich **S_{AB}** und **S_{BB}** in der Rektifikationskolonne **RD_{A}** vermischen, so dass in jedem Fall nach Durchführung von Schritt (b) eine Auftrennung in einen ROH umfassenden Brüdenstrom **S_{B2}** am Kopf von **RD_{A}** und einen Wasserstrom **S_{W}** am Sumpf von **RD_{A}** erfolgt.

Der Druck, den der Brüdenstrom **S_{B2}** aufweist, wird mit **"p_{B2}",** seine Temperatur mit **"T_{B2}"** bezeichnet. Dies bezieht sich insbesondere auf Druck und Temperatur von **S_{B2},** bevor **S_{B2}** dem Schritt (c) des erfindungsgemäßen Verfahrens unterworfen wird.

Bevorzugt wird in Schritt (b) auch der Wasserstrom **S_{W}** am Sumpf der Rektifikationskolonne **RD_{A}** entnommen.

Als Rektifikationskolonne **RD_{A}** in Schritt (b) des erfindungsgemäßen Verfahrens kann jede beliebige, dem Fachmann bekannte Rektifikationskolonne eingesetzt werden. Bevorzugt enthält die Reaktionskolonne **RD_{A}** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne möglichst gering bleibt und somit die mechanische Leistung des Verdichters und die Temperatur des zu verdampfenden Alkohol/Wasser-Gemischs gering bleibt.

Wenn in der Rektifikationskolonne **RD_{A}** strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Brüdenstroms **S_{AB}** bzw. der Zulaufstellen der beiden Brüdenströme **S_{AB}** und **S_{BB}** und eine Packung unterhalb der Zulaufstelle des Brüdenstroms **S_{AB}** bzw. der Zulaufstellen der beiden Brüdenströme **S_{AB}** und **S_{BB}** . Wenn eine unstrukturierte Packung eingesetzt wird, beispielsweise eine Füllkörperpackung, so liegen die Füllkörper üblicherweise auf einem geeigneten Siebboden oder Gitterboden auf.

Im Verbund aus Reaktionskolonne **RR_{A}** (bzw. in den Ausführungsformen, in denen Schritt (a2) durchgeführt wird, Reaktionskolonne **RR_{A}** und Reaktionskolonne **RR_{B})** mit Rektifikationskolonne **RD_{A}** im erfindungsgemäßen Verfahren wird die Rektifikationskolonne **RD_{A}** vorzugsweise bei einem Druck betrieben, der so gewählt wird, dass das Druckgefälle zwischen den Kolonnen im Falle der Brüdenverdichtung gemäß Schritt (c) mit geringem Aufwand möglich ist.

Im erfindungsgemäßen Verfahren wird der Alkohol ROH verbraucht, und insbesondere bei kontinuierlicher Verfahrensführung muss dieser deshalb mit frischem Alkohol ROH ersetzt werden.

Die Zuführung des frischen Alkohols ROH erfolgt dabei insbesondere direkt als Eduktstrom **S_{AE1}** umfassend ROH in die Reaktionskolonne **RR_{A}** bzw. in den Ausführungsformen, in denen Schritt (a2) durchgeführt wird, in die Reaktionskolonnen **RR_{A}** und **RR_{B}.**

Im erfindungsgemäßen Verfahren ist es weiterhin bevorzugt, den ROH umfassende Brüdenstrom **S_{B2}** teilweise als Eduktstrom **S_{AE1}** in Schritt (a1) und gegebenenfalls als Eduktstrom **S_{BE1}** in Schritt (a2) einzusetzen. Alternativ oder zusätzlich kann der verdichtete Brüdenstrom **S_{B3}** teilweise als Eduktstrom **S_{AE1}** in Schritt (a1) und gegebenenfalls als Eduktstrom **S_{BE1}** in Schritt (a2) eingesetzt werden. In dieser bevorzugten Ausführungsform ist es noch bevorzugter, wenn der frische Alkohol ROH der Rektifikationskolonne **RD_{A}** zugegeben wird.

Wenn der frische Alkohol ROH der Rektifikationskolonne **RD_{A}** zugegeben wird, wird er bevorzugt entweder im Verstärkungsteil der Rektifikationskolonne **RD_{A}** oder direkt am Kopf der Rektifikationskolonne **RD_{A}** zugeführt. Die optimale Zulaufstelle ist abhängig vom Wassergehalt des eingesetzten frischen Alkohols und andererseits von dem gewünschten Restwassergehalt im Brüdenstrom **S_{B2}.** Je höher der Wasseranteil im eingesetzten Alkohol und je höher die Reinheitsanforderung im Brüdenstrom **S_{B2}** ist, um so günstiger ist ein Zulauf einige theoretische Böden unterhalb des Kopfes der Rektifikationskolonne **RD_{A}.** Bevorzugt sind bis zu 20 theoretische Böden unterhalb des Kopfes der Rektifikationskolonne **RD_{A}** und insbesondere 1 bis 5 theoretische Böden.

Wenn der frische Alkohol ROH der Rektifikationskolonne **RD_{A}** zugegeben wird, wird er bei Temperaturen bis hin zum Siedepunkt, bevorzugt bei Raumtemperatur, am Kopf der Rektifikationskolonne **RD_{A}** zugegeben. Hierbei kann für den frischen Alkohol ein eigener Zulauf vorgesehen sein oder aber bei Rückführung eines Teils des am Kopf der Rektifikationskolonne **RD_{A}** entnommenen Alkohols nach der Kondensation mit diesem gemischt und gemeinsam in die Rektifikationskolonne **RD_{A}** zugeführt werden. In diesem Fall ist es besonders bevorzugt, wenn der frische Alkohol in einen Kondensatbehälter, in dem der aus dem Brüdenstrom **S_{B2}** kondensierte Alkohol gesammelt wird, zugegeben wird.

In einer vorteilhaften Ausgestaltung der Erfindung sind die Reaktionskolonne **RR_{A}** gemäß Schritt (a1), bzw. Reaktionskolonnen **RR_{A}** und **RR_{B}** in der vorbeschriebenen bevorzugten Ausführungsform, in der Schritt (a2) durchgeführt wird, und die Rektifikationskolonne **RD_{A}** gemäß Schritt (b) zur Auftrennung des Brüdenstroms **S_{B1}** in einem Kolonnenmantel untergebracht, wobei die Kolonne mindestens teilweise durch mindestens eine Trennwand, bzw. in der vorbeschriebenen bevorzugten Ausführungsform, in der Schritt (a2) durchgeführt wird, durch mindestens zwei Trennwände, unterteilt ist, wobei die mindestens eine Trennwand bzw. die mindestens zwei Trennwände bis zum Boden der Kolonne durchgezogen sind. Wie unter Punkt 4.2 beschrieben, handelt es sich dann wieder um eine Trennwandkolonne.

In diesem Fall wird in einem Teil der TRD die Reaktion zum Rohprodukt **RP_{A}** gemäß Schritt (a1) bzw. den Rohprodukten **RP_{A}** und **RP_{B}** gemäß Schritten (a1) und (a2) durchgeführt, wobei der Eduktstrom **S_{AE2}** und gegebenenfalls der Eduktstrom **S_{BE2}** zwar unterhalb, aber in etwa auf der Höhe des oberen Endes der Trennwand zugegeben wird und der Eduktstrom **S_{AE1}** und gegebenenfalls der Eduktstrom **S_{BE1}** dampfförmig am unteren Ende aufgegeben wird. Das oberhalb der Zugabestelle des Eduktstroms entstehende Alkohol/Wasser-Gemisch verteilt sich dann oberhalb der Trennwand über den gesamten Kolonnenbereich, der als Verstärkungsteil der Rektifikationskolonne **RD_{A}** dient. Der zweite, durch die Trennwand abgetrennte untere Teil der Kolonne ist der Abtriebsteil der Rektifikationskolonne **RD_{A}.** Die für die Destillation notwendige Energie wird dann über einen Verdampfer am unteren Ende des zweiten durch die Trennwand abgetrennten Teils der Kolonne zugeführt, wobei dieser Verdampfer konventionell beheizt werden kann oder mit dem verdichteten Brüdenstrom **S_{B3}** beheizt werden kann. Wenn der Verdampfer konventionell beheizt wird, so kann zusätzlich ein Zwischenverdampfer vorgesehen werden, der mit dem verdichteten Brüdenstrom **S_{B3}** beheizt wird.

Im Anschluss an Schritt (b) des erfindungsgemäßen Verfahrens wird dann der Brüdenstrom **S_{B2}** umfassend ROH am Kopf der Rektifikationskolonne **RD_{A}** entnommen. Der bevorzugte Massenanteil von ROH in diesem Brüdenstrom **S_{B2}** ist ≥ 99 Gew.-%, bevorzugt ≥ 99.6 Gew.-%, bevorzugter ≥ 99.9 Gew.-%, wobei der Rest insbesondere Wasser ist.

Am Sumpf von **RD_{A}** wird ein Wasserstrom **S_{W}** erhalten, der < 1 Gew.-% Alkohol aufweisen kann.

Die Entnahme des ROH umfassenden Brüdenstroms **S_{B2}** am Kopf der Rektifikationskolonne **RD_{A}** bedeutet im Rahmen der vorliegenden Erfindung, dass der Brüdenstrom **S_{B2}** als Kopfstrom oder als Seitenabzug oberhalb der Einbauten in der Rektifikationskolonne **RD_{A}** entnommen wird. Die bevorzugte Entnahme des im Wesentlichen Wasser enthaltenden Stroms **S_{W}** am Sumpf der Rektifikationskolonne **RD_{A}** erfolgt üblicherweise am unteren Boden der Rektifikationskolonne **RD_{A}** , jedoch kann die Entnahme auch über einen Seitenabzug im Sumpf erfolgen.

### 4.4 Schritt (c) des erfindungsgemäßen Verfahrens

Im Schritt (c) des erfindungsgemäßen Verfahrens wird mindestens eines Teils der Brüdenstroms **S_{B2}** verdichtet. Dadurch wird ein gegenüber **S_{B2}** verdichteter Brüdenstrom **S_{B3}** erhalten. Der Druck, den der Brüdenstrom **S_{B3}** aufweist, wird mit **"p_{B3}",** seine Temperatur mit **"T_{B3}"** bezeichnet.

Der Druck **p_{B3}** von **S_{B3}** ist höher, bevorzugt 1 bis 10 bar höher, bevorzugter 3 bis 6 bar höher, noch bevorzugter 5 bar höher, als der Druck **p_{B2}** von **S_{B2}** und die Temperatur **T_{B3}** von **S_{B3}** ist höher, bevorzugt 10 bis 150 °C, bevorzugter 50 bis 100 °C, noch bevorzugter 95 °C, als die Temperatur **T_{B2}** von **S_{B2}.**

Das Verdichten des Brüdenstroms **S_{B2}** in Schritt (c) kann auf jede beliebige, dem Fachmann bekannte Art erfolgen. So kann die Verdichtung zum Beispiel einstufig oder mehrstufig, bevorzugt mehrstufig, durchgeführt werden. Bei einer mehrstufigen Verdichtung können mehrere Verdichter gleicher Bauart oder Verdichter unterschiedlicher Bauart eingesetzt werden. Der Einsatz einer einstufigen Verdichtung oder einer mehrstufigen Verdichtung ist davon abhängig, auf welchen Druck der Brüden **S_{B2}** verdichtet werden soll.

In einer bevorzugten Ausführungsform des erfindungsgemäßen Verfahrens werden 10 bis 90 Gew.-%, bevorzugt 20 bis 80 Gew.-%, bevorzugter 30 bis 70 Gew.-%, noch bevorzugter 40 bis 60 Gew.-%, noch mehr bevorzugter 50 Gew.-% des Brüdenstromes **S_{B2}** als Eduktstrom **S_{AE1}** bzw. wenn Schritt (a2) durchgeführt wird, als Eduktstrom **S_{AE1}** und/oder Eduktstrom **S_{BE1}** eingesetzt.

Dazu ist es vorteilhaft, wenn zumindest der Teil, der als Eduktstrom **S_{AE1}** und/oder Eduktstrom **S_{BE1}** eingesetzt wird, mit einem ersten Verdichter **VD_{AB2}** verdichtet ("vorverdichtet") wird. Alternativ oder zusätzlich kann in dieser bevorzugten Ausführungsform auch der Verdichter **VD_{AB1}** wie oben beschrieben eingesetzt werden, um diese Vorverdichtung zu gewährleisten.

Der übrige, also nicht als Eduktstrom **S_{AE1}** und/oder Eduktstrom **S_{BE1}** eingesetzte Teil des Brüdenstromes **S_{B2}** wird in dieser bevorzugten Ausführungsform dann weiter verdichtet und im erfindungsgemäßen Schritt (d) eingesetzt. Diese zusätzliche Verdichtung wird insbesondere mit mindestens einem gegenüber **VD_{AB1}** und **VD_{AB2}** zusätzlichen Verdichter **VD,** durchgeführt.

Die Temperatur T_{B3} und der Druck p_{B3}, auf den S_{B3} in Schritt (c) verdichtet wird, wird dabei eingestellt in Abhängigkeit davon, auf welchen von **RP_{A}, S_{AE1}, S_{AE2}, S_{AP}, S_{AB}** in Schritt (a1), bzw., wenn Schritt (a2) durchgeführt wird, auf welchen von **RP_{A}, S_{AE1}, S_{AE2}, S_{AP}, S_{AB}, RP_{B}, S_{BE1}, S_{BE2}, S_{BP}, S_{BB}** die Energie übertragen werden soll. Es versteht sich von selbst, dass, wenn mit **S_{B3}** beispielsweise eine dieser Komponenten beheizt werden soll, dass dann am Ende des Schrittes (c) die Temperatur **T_{B3}** von **S_{B3}** höher als die Temperatur der in Schritt (d) zu beheizenden Komponente sein muss. Dies kann vom Fachmann gemäß seinem Fachwissen eingestellt werden. Das Entsprechende gilt für den Druck **p₃**, auf welchen **S_{B3}** eingestellt werden soll. Auch dieser kann in Abhängigkeit der Anforderungen in Schritt (d) vom Fachmann eingestellt werden.

Wenn Energie des verdichteten Brüdenstrom **S_{B3}** beispielsweise auf das Rohprodukt **RP_{A}** übertragen werden soll und insbesondere das Rohprodukt **RP_{A}** über einen Zwischenverdampfer V_{ZA} beheizt werden soll, der an der Reaktionskolonne **RR_{A}** angebracht ist, muss zum Beheizen des Zwischenverdampfers **V_{ZA}** eine kleinere Druckdifferenz überwunden werden, als wenn Energie des Brüdenstroms **S_{B3}** zum Beheizen eines Verdampfers **V_{SA}** am Sumpf der Reaktionskolonne **RR_{A},** mit dem dann **S_{AP}** erhitzt wird, in Schritt (d) des erfindungsgemäßen Verfahrens eingesetzt wird.

Die größere zu überwindende Druckdifferenz kann durch zusätzliche Verdichterstufen oder durch einen stärkeren Verdichter überwunden werden. Üblicherweise werden jedoch zusätzliche Verdichterstufen eingesetzt.

Als Verdichter im erfindungsgemäßen Verfahren, insbesondere zum Verdichten des Brüdenstroms **S_{B2},** eignet sich jeder beliebige, dem Fachmann bekannte Verdichter, mit dem sich Gasströme verdichten lassen. Geeignete Verdichter sind zum Beispiel ein- oder mehrstufige Turbinen, Kolbenverdichter, Schraubenverdichter, Zentrifugalverdichter oder Axialverdichter.

Bei einer mehrstufigen Verdichtung werden für die jeweils zu überwindenden Druckstufen geeignete Verdichter eingesetzt.

### 4.5 Schritt (d) des erfindungsgemäßen Verfahrens

Im Schritt (d) des erfindungsgemäßen Verfahrens wird Energie von **S_{B3}** auf mindestens einen von **RP_{A}, S_{AE1}, S_{AE2}, S_{AP}, S_{AB},** bevorzugt mindestens einen von **RP_{A}, S_{AP},** noch bevorzugter **S_{AP},** übertragen. In der optionalen Ausführungsform des erfindungsgemäßen Verfahrens, in der Schritt (a2) durchgeführt wird, wird alternativ oder zusätzlich, bevorzugt zusätzlich, Energie von **S_{B3}** auf mindestens einen von **RP_{B}, S_{BE1}, S_{BE2}, S_{BP}, S_{BB},** bevorzugt mindestens einen **RP_{B}, S_{BP},** bevorzugter **S_{BP}** übertragen.

Wenn Schritt (a2) durchgeführt wird, wird demnach in Schritt (d) des erfindungsgemäßen Verfahrens Energie von **S_{B3}** auf mindestens einen von **RP_{A}, S_{AE1}, S_{AE2}, S_{AP}, S_{AB}, RP_{B}, S_{BE1}, S_{BE2}, S_{BP}, S_{BB},** bevorzugt mindestens einen von **RP_{A}, S_{AP}, RP_{B}, S_{BP},** bevorzugt auf sowohl S_{AP} als auch **S_{BP}** übertragen.

Dadurch wird ein Brüdenstrom **S_{B4}** mit einem geringeren Energiegehalt als **S_{B3}** erhalten. Dies bedeutet insbesondere, dass für den Druck **p_{B4}** von **S_{B4}** und die Temperatur **T_{B4}** von **S_{B4}** gilt: **p_{B4}** ≤ **p_{B3}** und **T_{B4}** < **T_{B3}.** Noch bevorzugter gilt, dass **T_{B2}** < **T_{B4}** und **p_{B2}** < **p_{B4},** so dass dann **T_{B2}** < **T_{B4}** < **T_{B3}** und **p_{B2}** < **p_{B4}** ≤ **p_{B3}.**

Dies bedeutet insbesondere, dass mit **S_{B3}** mindestens eines von **RP_{A}, S_{AE1}, S_{AE2}, S_{AP}, S_{AB},** bevorzugt mindestens einen von **RP_{A}, S_{AP},** noch bevorzugter **S_{AP}** beheizt wird.

In der optionalen Ausführungsform des erfindungsgemäßen Verfahrens, in der Schritt (a2) durchgeführt wird, bedeutet dies insbesondere, dass alternativ oder zusätzlich, bevorzugt zusätzlich, mit **S_{B3}** mindestens einer von **RP_{B}, S_{BE1}, S_{BE2}, S_{BP}, S_{BB},** bevorzugt mindestens einer von **RP_{B}, S_{BP},** bevorzugt **S_{BP}** beheizt wird.

Wenn Schritt (a2) durchgeführt wird, wird noch bevorzugter mit **S_{B3}** mindestens einer von **RP_{A}, S_{AE1}, S_{AE2}, S_{AP}, S_{AB}, RP_{B}, S_{BE1}, S_{BE2}, S_{BP}, S_{BB},** bevorzugt mindestens einer von **RP_{A}, S_{AP}, RP_{B}, S_{BP},** bevorzugt sowohl **S_{AP}** als auch **S_{BP},** beheizt.

Somit ist die Temperatur **T_{B3}** von **S_{B3}** nach Durchführung des Schrittes (c) des erfindungsgemäßen Verfahrens bevorzugt höher als derjenigen Komponente ausgewählt aus **RP_{A}, S_{AE1}, S_{AE2}, S_{AP}, S_{AB}, RP_{B}, S_{BE1}, S_{BE2}, S_{BP}, S_{BB},** die in Schritt (d) durch **S_{B3}** beheizt wird.

Die Übertragung von Energie auf, bevorzugt die Beheizung von, mindestens eines von **RP_{A}, S_{AE1}, S_{AE2}, S_{AP}, S_{AB}, RP_{B}, S_{BE1}, S_{BE2}, S_{BP}, S_{BB}** durch **S_{B3}** erfolgt bevorzugt direkt oder indirekt.

"Direkt" bedeutet, dass **S_{B3}** mit der mindestens einer Komponente ausgewählt aus **RP_{A}, S_{AE1}, S_{AE2}, S_{AP}, S_{AB}** bzw. wenn Schritt (a2) durchgeführt wird, ausgewählt aus **RP_{A}, S_{AE1}, S_{AE2}, S_{AP}, S_{AB}, RP_{B}, S_{BE1}, S_{BE2}, S_{BP}, S_{BB}** kontaktiert wird, so dass Wärme von **S_{B3}** auf die betreffende mindestens eine Komponente übergeht.

Im Falle von **S_{AP},** S_{BP} kann dies durchgeführt werden, in dem **S_{B3}** mindestens teilweise durch einen Sumpfverdampfer **V_{SA}** bzw. **V_{SB}** an der Reaktionskolonne **RR_{A}** bzw. **RR_{B}** geleitet wird und dort **S_{AP}** bzw. **S_{BP}** beheizt.

Als Wärmetauscher können die dem Fachmann geläufigen Wärmetauscher eingesetzt werden.

"Indirekt" bedeutet insbesondere, dass **S_{B3}** mit mindestens einem Wärmeträger **W₁,** bevorzugt über mindestens einen Wärmetauscher **WTₓ,** kontaktiert wird, wobei es sich bei dem Wärmeträger nicht um **RP_{A}, S_{AE1}, S_{AE2}, S_{AP}, S_{AB}, RP_{B}, S_{BE1}, S_{BE2}, S_{BP}** oder **S_{BB}** handelt, er also von diesen verschieden ist, so dass Energie, bevorzugt Wärme, von **S_{B3}** auf den mindestens einen Wärmeträger **W,** übergeht, und die Wärme dann von **W₁** auf die betreffende mindestens eine Komponente ausgewählt aus **RP_{A}, S_{AE1}, S_{AE2}, S_{AP}, S_{AB}, RP_{B}, S_{BE1}, S_{BE2}, S_{BP}, S_{BB}** übergeht, in dem W, die betreffende Komponente kontaktiert.

In einer weiteren Ausführungsform des erfindungsgemäßen Verfahrens kann bei indirekter Beheizung von mindestens einem von **RP_{A}, S_{AE1}, S_{AE2}, S_{AP}, S_{AB}, RP_{B}, S_{BE1}, S_{BE2}, S_{BP}, S_{BB}** mit **S_{B3}** auch zunächst Wärme von **S_{B3} auf W₁,** bevorzugt durch Kontaktierung über mindestens einen Wärmetauscher **WT_{X}** übertragen werden, und dann von **W₁** auf einen weiteren, von **RP_{A},** S_{AE1}, **S_{AE2}, S_{AP}, S_{AB}, RP_{B}, S_{BE1}, S_{BE2}, S_{BP}, S_{BB}** verschiedenes Wärmeträger **W₂,** bevorzugt durch Kontaktierung über mindestens einen Wärmetauscher **WT_{Y},** übertragen werden. Im letzten Schritt erfolgt dann Übertragung der Wärme von **W₂** auf mindestens einen von **RP_{A}, S_{AE1}, S_{AE2}, S_{AP}, S_{AB}, RP_{B}, S_{BE1}, S_{BE2}, S_{BP}, S_{BB}.** Es versteht sich von selbst, dass sich in weiteren Ausführungsformen der vorliegenden Erfindung dementsprechend noch weitere Wärmeträger **W₃, W₄, W₅** etc. nutzen lassen.

Als Wärmeträger **W,** bzw. daneben weiter genutzte Wärmeträger **W₂, W₃, W₄, W₅** kann jeder dem Fachmann bekannte Wärmeträger genutzt werden, bevorzugt sind sie ausgewählt aus der Gruppe bestehend aus Wasser; Alkohol-Wasser-Lösungen; Salz-Wasser-Lösungen, wozu auch ionische Flüssigkeiten, wie zum Beispiel LiBr-Lösungen, Dialkylimidazoliumsalze wie insbesondere Dialkylimidazoliumdialkylphosphate, gehören; Mineralöle, wie zum Beispiel Dieselöle; Thermalöle wie zum Beispiel Silikonöle; biologische Öle wie zum Beispiel Limonen; aromatische Kohlenwasserstoffe wie zum Beispiel Dibenzyltoluol. Am bevorzugtesten ist als Wärmeträger **W,** Wasser.

Salz-Wasser-Lösungen, die verwendet werden können, sind beispielsweise auch in der DE 10 2005 028 451 A1 und der WO 2006/134015 A1 beschrieben.

Die Übertragung von Energie von S_{B3} auf das Rohprodukt **RP_{A}, RP_{B}** und/oder mindestens einen Strom ausgewählt aus **S_{AE1}, S_{AE2}, S_{AP}, S_{AB}, S_{BE1}, S_{BE2}, S_{BP}, S_{BB}** erfolgt nach dem Fachmann bekannten Verfahren, und bevorzugt nach einem der im Folgenden unter den Nummern 4.5.1, 4.5.2, 4.5.3 und 4.5.4 genannten Ausführungsformen, wobei die unter 4.5.4 genannte am bevorzugtesten ist.

4.5.1) In der Ausführungsform der vorliegenden Erfindung, in der in Schritt (d) Energie von **S_{B3}** auf mindestens eines von **RP_{A}** und/oder **RP_{B}** übertragen wird, erfolgt dies bevorzugt dergestalt, dass an der Reaktionskolonne **RR_{A}** bzw. **RR_{B}** mindestens ein Zwischenverdampfer **V_{ZA}** bzw. **V_{ZB}** angebracht ist, mit Hilfe dessen flüssiges Rohprodukt **RP_{A}** bzw. **RP_{B}** innerhalb der Reaktionskolonne **RR_{A}** bzw. **RR_{B}** verdampft wird. In dieser bevorzugten Ausführungsform wird insbesondere dieser Zwischenverdampfer **V_{ZA}** bzw. **V_{ZB}** mit **S_{B3}** beheizt, falls eine direkte Beheizung erfolgt, oder, falls eine indirekte Beheizung erfolgt, mit einem Wärmeträger **W₁,** bei dem es sich nicht um S_{B3} handelt, beheizt, wobei der Wärmeträger **W,** davor Energie von **S_{B3}** aufgenommen hat.

4.5.2) In der Ausführungsform der vorliegenden Erfindung, in der in Schritt (d) Energie von **S_{B3}** auf den mindestens einen Eduktstrom ausgewählt aus **S_{AE1}, S_{AE2}, S_{BE1}, S_{BE2}** übertragen wird, erfolgt dies bevorzugt dergestalt, dass der jeweilige Eduktstrom, bevor er in die Reaktionskolonne **RR_{A}** bzw. **RR_{B}** geleitet wird, mindestens einen Wärmetauscher **WT1** passiert. In **WT1** erfolgt die Übertragung von Energie, bevorzugt Wärme, von **S_{B3},** falls eine direkte Beheizung erfolgt, oder, falls eine indirekte Beheizung erfolgt, von einem Wärmeträger **W₁,** bei dem es sich nicht um **S_{B3}** handelt, wobei der Wärmeträger **W₁** davor Energie von **S_{B3}** aufgenommen hat, auf mindestens einen Eduktstrom **S_{AE1}, S_{AE2}, S_{BE1}, S_{BE2}.**

4.5.3) In der Ausführungsform der vorliegenden Erfindung, in der in Schritt (d) Energie von **S_{B3}** auf mindestens einen Brüdenstrom ausgewählt aus **S_{AB}, S_{BB}** übertragen wird, erfolgt dies bevorzugt dergestalt, dass der jeweilige Brüdenstrom, bevor er in die Rektifikationskolonne **RD_{A}** geleitet wird, einen Wärmetauscher **WT2** passiert. In **WT2** erfolgt die Übertragung von Energie, bevorzugt Wärme, von **S_{B3},** falls eine direkte Beheizung erfolgt, oder, falls eine indirekte Beheizung erfolgt, von einem Wärmeträger **W₁,** bei dem es sich nicht um **S_{B3}** handelt, wobei der Wärmeträger **W₁** davor Energie von **S_{B3}** aufgenommen hat, auf den mindestens einen Brüdenstrom ausgewählt aus **S_{AB}**, **S_{BB}**.

4.5.4) In der Ausführungsform der vorliegenden Erfindung, in der in Schritt (d) Energie direkt von **S_{B3}** auf mindestens einen Sumpfproduktstrom ausgewählt aus **S_{AP}, S_{BP}** übertragen wird, erfolgt dies vorteilhafterweise dergestalt, dass an der Reaktionskolonne **RR_{A}** bzw. **RR_{B}** mindestens ein Sumpfverdampfer **V_{SA}** bzw. **V_{SB}** angebracht ist, über den **S_{B3}** mindestens teilweise geleitet wird, und über den **S_{AP}** bzw. **S_{BP}** nach Verlassen der Reaktionskolonne **RR_{A}** bzw. **RR_{B}** mindestens teilweise geleitet werden, so dass Energie, bevorzugt Wärme, von **S_{B3}** auf **S_{AP}** bzw. **S_{BP}** übertragen wird, insbesondere so dass **S_{AP}** bzw. **S_{BP}** mindestens teilweise verdampft wird, insbesondere ROH aus **S_{AP}** bzw. **S_{BP}** mindestens teilweise verdampft wird, wodurch ein Sumpfproduktstrom **S_{AP*}** bzw. **S_{BP*}** erhalten wird.

In der Ausführungsform der vorliegenden Erfindung, in der in Schritt (d) Energie indirekt von **S_{B3}** auf mindestens einen Sumpfproduktstrom ausgewählt aus **S_{AP}, S_{BP}** übertragen wird, erfolgt dies vorteilhafterweise dergestalt, dass **S_{B3},** bevorzugt über einen Wärmetauscher, einen von **RP_{A}, S_{AE1}, S_{AE2}, S_{AP}, S_{AB}, RP_{B}, S_{BE1}, S_{BE2}, S_{BP}** oder **S_{BB}** verschiedenen Wärmeträger **W₁,** bevorzugt Wasser, kontaktiert, so dass Energie, bevorzugt Wärme, von **S_{B3}** auf den mindestens einen Wärmeträger **W₁** übergeht,
und an der Reaktionskolonne **RR_{A}** bzw. **RR_{B}** mindestens ein Sumpfverdampfer **V_{SA}** bzw. **V_{SB}** angebracht ist, über den **W₁** mindestens teilweise geleitet wird, und über den **S_{AP}** bzw. **S_{BP}** nach Verlassen der Reaktionskolonne **RR_{A}** bzw. **RR_{B}** mindestens teilweise geleitet werden, so dass Energie, bevorzugt Wärme, von **W₁** auf **S_{AP}** bzw. **S_{BP}** übertragen wird, insbesondere so dass **S_{AP}** bzw. **S_{BP}** mindestens teilweise verdampft wird, insbesondere ROH aus **S_{AP}** bzw. **S_{BP}** mindestens teilweise verdampft wird, wodurch ein Sumpfproduktstrom **S_{AP*}** bzw. **S_{BP*}** erhalten wird.

In der bevorzugten Ausführungsform gemäß 4.5.4, ist es noch bevorzugter, dass zusätzlich zu dem Verdampfer **V_{SA}** bzw. **V_{SB},** mindestens ein weiterer, konventionell beheizter Verdampfer **V_{KA}** and der Reaktionskolonne **RR_{A}** bzw. **V_{KB}** and der Reaktionskolonne **RR_{B}** eingesetzt wird, der am Sumpf der Reaktionskolonne **RR_{A}** bzw. **RR_{B}** angeordnet ist. Wenn der mit dem Brüdenstrom **S_{B3}** direkt oder indirekt beheizte Verdampfer **V_{SA}** bzw. **V_{SB}** ebenfalls am Sumpf der Reaktionskolonne **RR_{A}** bzw. **RR_{B}** angeordnet ist, kann der konventionell beheizte Verdampfer **V_{KA}** bzw. **V_{KB}** zum Beispiel dazu genutzt werden, während des Betriebes der Reaktionskolonne **RR_{A}** bzw. **RR_{B}** zusätzlich Wärme zuzuführen. Im Allgemeinen wird der konventionell beheizte Verdampfer **V_{KA}** bzw. **V_{KB}** jedoch dazu genutzt, die Reaktionskolonne **RR_{A}** bzw. **RR_{B}** anzufahren. Beim Anfahren der Reaktionskolonne **RR_{A}** bzw. **RR_{B}** steht noch nicht ausreichend Brüden **S_{B3}** zur Verfügung, mit dem der Verdampfer **V_{SA}** bzw. **V_{SB}** direkt oder indirekt beheizt werden kann, so dass zunächst von außerhalb Wärme zugeführt werden muss. Nach dem Anfahren der Rektifikationskolonne **RD_{A}** nimmt die Brüdenmenge **S_{B2},** die am Kopf der Rektifikationskolonne **RD_{A}** entnommen werden kann, zu. Diese kann dann zu **S_{B3}** verdichtet werden, und der Betrieb kann auf den mit dem verdichteten Brüdenstrom **S_{B3}** beheizten Verdampfer **V_{SA}** bzw. **V_{SB}** umgestellt werden. Hierbei ist es einerseits möglich, den Verdampfer **V_{SA}** bzw. **V_{SB},** der mit dem Brüdenstrom **S_{B3}** beheizt wird, langsam in Betrieb zu nehmen und den konventionell beheizten Verdampfer **V_{KA}** bzw. **V_{KB}** entsprechend weniger zu beheizen oder es wird gewartet, bis sich ein stationärer Betriebszustand in der Rektifikationskolonne **RR_{A}** bzw. **RR_{B}** eingestellt hat, und dann vom konventionell beheizten Verdampfer **V_{KA}** bzw. **V_{KB}** auf den mit dem verdichteten Brüdenstrom **S_{B3}** beheizten Verdampfer **V_{SA}** bzw. **V_{SB}** umgeschaltet.

Wenn, beispielsweise wie in der unter Punkt 4.5.1 beschriebenen Ausführungsform der vorliegenden Erfindung, mit dem verdichteten Brüdenstrom **S_{B3}** ein Zwischenverdampfer **V_{ZA}** bzw. **V_{ZB}** beheizt wird, wird der zusätzliche, konventionell beheizte Verdampfer **V_{KA}** bzw. **V_{KB}** genutzt, um weitere Wärme am Sumpf der Reaktionskolonne **RR_{A}** bzw. **RR_{B}** in die Reaktionskolonne **RR_{A}** bzw. **RR_{B}** einzubringen. In diesem Fall wird der konventionell beheizte Verdampfer **V_{KA}** bzw. **V_{KB}** über die gesamte Betriebsdauer der Reaktionskolonne **RR_{A}** bzw. **RR_{B}** betrieben. Auch hier muss zum Anfahren der Reaktionskolonne **RR_{A}** bzw. **RR_{B}** zunächst über den konventionell beheizten Verdampfer **V_{KA}** bzw. **V_{KB}** eine größere Wärmemenge in die Reaktionskolonne **RR_{A}** bzw. **RR_{B}** eingebracht werden, bis der Zwischenverdampfer **V_{ZA}** bzw. **V_{ZB}** mit einem ausreichend großen Brüdenstrom **S_{B3}** versorgt werden kann, um diesen zu beheizen. Dann kann die Wärmemenge, die mit dem konventionell beheizten Verdampfer **V_{KA}** bzw. **V_{KB}** in die Reaktionskolonne **RR_{A}** bzw. **RR_{B}** eingebracht wird, verringert werden. Alternativ ist es bei Einsatz eines Zwischenverdampfers **V_{ZA}** bzw. **V_{ZB}** auch möglich, zwei konventionell beheizte Verdampfer **V_{KA1}** und **V_{KA2}** bzw. **V_{KB1}** und **V_{KB2}** am Sumpf der jeweiligen Reaktionskolonne **RR_{A}** bzw. **RR_{B}** einzusetzen. Der zusätzliche konventionell beheizte Verdampfer **V_{KA2}** bzw. **V_{KB2}** wird dann zum Anfahren der Kolonne genutzt und der andere konventionell beheizte Verdampfer **V_{KA1}** bzw. **V_{KB1}** wird während des Betriebs der Reaktionskolonne **RR_{A}** bzw. **RR_{B}** weiter betrieben.

Der Schritt (d) des erfindungsgemäßen Verfahrens spiegelt den unerwarteten Effekt der vorliegenden Erfindung wider. Mit dem erfinderischen Verfahren ist es möglich, die bei der Verdichtung des Brüdenstrom **S_{B2}** zum verdichteten Brüdenstrom **S_{B3}** erhaltene überschüssige Energie nicht ungenutzt dissipieren zu lassen, sondern sie zum Betreiben der Umsetzung in der Reaktivdestillation einzusetzen. Dies führt zu einer deutlichen Einsparung an Energie.

### 4.6 Bevorzugter Schritt (e) des erfindungsgemäßen Verfahrens

In einer bevorzugten Ausführungsform des erfindungsgemäßen Verfahrens wird in einem Schritt (e) der Brüdenstrom **S_{B4}** zur Beheizung der Rektifikationskolonne **RD_{A}** eingesetzt. Dies kann insbesondere dadurch gewährleistet werden, dass an deren unterem Ende ein Sumpfverdampfer **V_{SRD}** angebracht ist, der den Wasserstrom **S_{W}** am Sumpf von **RD_{A}** beheizt. Der Wasserstrom **S_{W}** kann so mindestens teilweise wieder in die Rektifikationskolonne **RD_{A}** rückgeführt werden. Alternativ oder zusätzlich, bevorzugt zusätzlich, kann der Brüdenstrom **S_{B4}** auch wieder, bevorzugt am Kopf der Rektifikationskolonne **RD_{A}**, in diese zurückgeführt werden. Wenn der Brüdenstrom **S_{B4}** in die Rektifikationskolonne **RD_{A}** zurückgeführt wird, wird er bevorzugt kondensiert und bei Temperaturen bis hin zum Siedepunkt, bevorzugt bei Raumtemperatur, bevorzugt am Kopf der Rektifikationskolonne **RD_{A}** zugegeben. Hierbei kann für den Brüdenstrom **S_{B4}** ein eigener Zulauf vorgesehen sein oder aber bei Rückführung eines Teils des am Kopf der Rektifikationskolonne **RD_{A}** entnommenen Alkohols nach der Kondensation mit diesem gemischt und gemeinsam in die Rektifikationskolonne **RD_{A}** zugeführt werden. In diesem Fall ist es besonders bevorzugt, wenn der Brüdenstrom **S_{B4}** in einen Kondensatbehälter, in dem der aus dem Brüdenstrom **S_{B2}** kondensierte Alkohol gesammelt wird, zugegeben wird.

Wird der Brüdenstrom **S_{B4}** in Schritt (e) eingesetzt, ist es vorteilhaft, ihn in mindestens einem weiteren Verdichter **VDₓ** zu verdichten, wodurch ein gegenüber **S_{B4}** verdichteter Brüdenstrom **S_{B5}** erhalten wird.

### 5. Bevorzugte Ausführungsformen der Erfindung

In Abbildung 1 ist eine Ausführungsform <10> des erfindungsgemäßen Verfahrens gezeigt. Es umfasst eine Reaktionskolonne **RR_{A}** <100>, die optional Stufen oberhalb des Eduktstroms **S_{AE2}** <102> umfassen kann. Am Kopf der Reaktionskolonne **RR_{A}** <100> wird eine wässrige NaOH-Lösung als Eduktstrom **S_{AE2}** <102> zugegeben. Alternativ kann auch eine methanolische KOH-Lösung als Eduktstrom **S_{AE2}** <102> zugegeben werden, um dann das entsprechende Kaliummethylat herzustellen. Oberhalb des Sumpfes der Reaktionskolonne **RR_{A}** <100> wird Methanol als Eduktstrom **S_{AE1}** <103> dampfförmig zugegeben. Am Sumpf der Reaktionskolonne **RR_{A}** <100> wird das Produkt Natriummethanolat gelöst in Methanol als Sumpfproduktstrom **S_{AP*}** <104> erhalten. Mit dem Sumpfverdampfer V_{SA} <105> und dem optionalen Verdampfer **V_{KA}** <106> am Sumpf der Kolonne **RR_{A}** <100> wird die Konzentration der Natriummethylatlösung **S_{AP*}** <104> auf den gewünschten Wert eingestellt. Der optionale Verdampfer V_{KA} <106> dient dabei insbesondere auch zum Anfahren der Kolonne **RR_{A}** <100>.

Am Kopf der Reaktionskolonne **RR_{A}** <100> wird ein Brüdenstrom **S_{AB}** <107> entnommen. Am Kopf der Reaktionskolonne **RR_{A}** <100> wird ein Teil des Brüdenstroms **S_{AB}** <107> im Kondensator **K_{RRA}** <108> kondensiert und flüssig als Rücklauf auf den Kopf der Reaktionskolonne **RR_{A}** <100> aufgegeben. Kondensator **K_{RRA}** <108> und die Einstellung des Rücklaufs sind jedoch optional.

Der Brüdenstrom **S_{AB}** <107> wird über die Leitung <13> einer Rektifikationskolonne **RD_{A}** <300> zugeleitet.

Am Kopf der Rektifikationskolonne **RD_{A}** <300> wird Methanolbrüden **S_{B2}** <302> abgeführt. Dieser wird dann über den Verdichter **VD_{AB2}** <303> geleitet und teilweise über Leitung <31> zur Reaktionskolonne **RR_{A}** <100> rezykliert, wo er als Eduktstrom **S_{AE1}** <103> eingesetzt wird. Alternativ oder zusätzlich zum Verdichter **VD_{AB2}** <303> kann auch der Verdichter **VD_{AB1}** <301> eingesetzt werden.

Die Erfindung besteht darin, dass mindestens ein Teil des Methanolbrüden **S_{B2}** <302>, nachdem er den Verdichter **VD_{AB2}** <303> passiert hat und dort vorverdichtet wurde, dem Verdichter **VD₁** <401> zugeleitet wird, wo er zum verdichteten Brüdenstrom **S_{B3}** <403> verdichtet wird. Der Brüdenstrom **S_{B3}** <403> beheizt den Sumpfverdampfer **V_{SA}** <105>. Dies erfolgt entweder durch direkte Beheizung von Sumpfverdampfer **V_{SA}** <105> durch **S_{B3}** <403> (nicht in Abbildung 1 dargestellt). Alternativ erfolgt die Beheizung wie in Abbildung 1 dargestellt, indem durch den Wärmetauscher **WTₓ** <402> Wärme an einen Wärmeträger (bevorzugt Wasser) **W₁** <410> übertragen wird, mit dessen Hilfe wiederrum der Sumpfverdampfer **V_{SA}** <105> beheizt wird. Der Wärmeträger (bevorzugt Wasser) **W₁** <410> zirkuliert in den Leitungen <41> und <14>, welche daneben auch optional Leitungen <141 > und <411> zur Regelung aufweisen können. Nach Durchlaufen des Wärmetauschers **WTₓ** <402> erhält man den Brüdenstrom **S_{B4}** <404> mit gegenüber **S_{B3}** <403> verringertem Energiegehalt. **S_{B4}** <404> durchläuft einen zusätzlichen Verdichter **VDₓ** <405> und wird dann als gegenüber **S_{B4}** <404> verdichteter Brüdenstrom **S_{B5}** <409> zum Beheizen des Sumpfverdampfers **V_{SRD}** <406> genutzt, wonach er über eine optionale Drossel <407> geführt wird und mit gegebenenfalls frischem Methanol <408> wieder der Rektifikationskolonne **RD_{A}** <300> als Rücklauf zugeführt wird. Am Sumpf der Rektifikationskolonne **RD_{A}** <300> wird ein Wasserstrom **S_{W}** <304> erhalten.

In Abbildung 2 ist eine weitere bevorzugte Ausführungsform der vorliegenden Erfindung gezeigt. Diese entspricht Abbildung 1, nur dass zwei Reaktionskolonnen **RR_{A}** <100A> und **RR_{B}** <100B> vorliegen, und die entsprechenden Bestandteile der Reaktionskolonnen **RR_{A}** <100A> und **RR_{B}** <100B> denen in Abbildung 1 für die Reaktionskolonne **RR_{A}** <100> angegebenen entsprechen, wobei jeder Bestandteil um den Zusatz "A" bzw. "B" erweitert ist, um die Zugehörigkeit zu **RR_{A}** bzw. **RR_{B}** zu kennzeichnen. In Reaktionskolonne **RR_{A}** <100A> wird ein Strom **S_{AE2}** <102A> umfassend NaOH zugegeben, in Reaktionskolonne **RR_{B}** <100B> wird ein Strom **S_{BE2}** <102B> umfassend KOH zugegeben. Beide Reaktionskolonnen **RR_{A}** <100A> und **RR_{B}** <100B> können oberhalb des Zulaufs der Alkalilauge zusätzliche Stufen aufweisen, dies ist jedoch optional und in Abbildung 2 nicht gezeigt. Im Unterschied zu Abbildung 1 weisen die Leitungen <14> und <41> Verzweigungen auf, die einen Wärmetausch des Brüden **S_{B3}**<403> mit beiden Verdampfern **V_{SA}** <105A> und **V_{SB}** <105B> ermöglichen. Dazu kann auch eine zusätzliche Drossel <412> zur Regelung optional eingefügt werden. Brüdenstrom **S_{AB}** <107A> und Brüdenstrom **S_{BB}** <107B> werden gemischt als Brüdenstrom <107C> der Rektifikationskolonne **RD_{A}** <300> zugeführt.

### 6. Beispiele

### Beispiel 1 (nicht erfindungsgemäß):

Ein Strom wässriger NaOH (50 Gew.-%) von 2 kg/h wird mit 30 °C am Kopf einer Reaktionskolonne zugeführt. Im Gegenstrom wird ein dampfförmiger Methanolstrom von 21 kg/h über dem Sumpf der Reaktionskolonne zugeführt. Die Reaktionskolonne wird bei einem Druck von 1.2 bar betrieben. Am Sumpf der Kolonne wird ein nahezu wasserfreier Produktstrom von 4.5 kg/h entnommen (30 Gew.-% Natriummethanolat in Methanol). Am Verdampfer der Reaktionskolonne werden ca. 0.5 kW Heizleistung mit Hilfe von Niederdruckdampf eingespeist. Ein dampfförmiger Methanol-Wasser-Strom von 18.5 kg/h wird am Kopf der Reaktionskolonne entnommen und einer Rektifikationskolonne zugeführt. Die Rektifikationskolonne wird bei ca. 1.1 bar betrieben. Am Sumpf der Rektifikationskolonne wird ein flüssiger Wasserstrom von 1.5 kg/h abgeführt. Am Kopf der Rektifikationskolonne wird ein dampfförmiger Methanolstrom von 33.4 kg/h entnommen, in einem Wärmetauscher auf 75 °C vorgewärmt und dann einem ersten Verdichter zugeführt. In diesem Verdichter wird der Strom auf 1.7 bar verdichtet. Anschließend wird der Strom aufgeteilt, ein Strom von 21 kg/h wird zu der Reaktionskolonne zurückgeführt. Der Rest von 12.4 kg/h wird einer zweistufigen Verdichtung mit Zwischenkühlung zugeführt. Im Verdichter 2 wird auf 4.9 bar und 180 °C verdichtet. In der anschließenden Zwischenkühlung wird der Strom auf ca. 150 °C abgekühlt, wobei ca. 0.2 kW an Wärme über Kühlwasser abgeführt werden. Im Verdichter 3 wird der Strom schließlich auf 6.2 bar und 170 °C verdichtet. Im nachfolgenden Kondensator, der Zugleich der Verdampfer der Rektifikationskolonne ist, werden die ca. 4 kW an Heizleistung für die Rektifikationskolonne zur Verfügung gestellt. Dem flüssigen Methanolstrom werden 3.9 kg/h Frischmethanol zugeführt und der gemischte Strom der Rektifikationskolonne am Kopf als Rücklauf aufgegeben.

### Beispiel 2 (erfindungsgemäß), entspricht Abbildung 1:

Identisch zu Beispiel 1, jedoch weist die Reaktionskolonne <100> zwei Sumpfverdampfer <105> und <106> auf. Im ersten Sumpfverdampfer <105> wird mit Hilfe eines Wärmeträgermediums der Wärmestrom von ca. 0.2 kW eingespeist, welcher in der Zwischenkühlung zwischen Verdichter 2 und 3 abgeführt und auf das Wärmeträgermedium (Wasser) übertragen wurde. Im zweiten Sumpfverdampfer wird der Rest der benötigten Energie über Niederdruckdampf bereitgestellt. Damit wird der Bedarf an Niederdruckdampf für die Reaktionskolonne <100> im Vergleich zu Beispiel 1 um etwa 40 % reduziert.

### Beispiel 3 (erfindungsgemäß), entspricht Abbildung 2:

Eine erste Reaktionskolonne wird identisch zu Beispiel 2 betrieben. Zusätzlich wird eine zweite Reaktionskolonne (im Folgenden "**K2**") mit wässriger KOH (50 Gew.-%) betrieben. Beide Reaktionskolonnen weisen wie in Beispiel 2 jeweils zwei Sumpfverdampfer auf. Ein Strom von 0.5 kg/h wird mit 30 °C am Kopf von **K2** zugeführt. Im Gegenstrom wird ein dampfförmiger Methanolstrom von 7.5 kg/h über dem Sumpf von **K2** zugeführt. **K2** wird bei einem Druck von 1.2 bar betrieben. Am Sumpf von **K2** wird ein nahezu wasserfreier Produktstrom von 1.5 kg/h entnommen (32 Gew.-% Kaliummethanolat in Methanol). In beiden Verdampfern von **K2** werden insgesamt ca. 0.2 kW Heizleistung eingespeist. Ein dampfförmiger Methanol-Wasser-Strom von 7 kg/h wird am Kopf von **K2** entnommen. Dieser Methanol-Wasser-Strom wird mit dem Methanol-Wasser-Strom der ersten Reaktionskolonne vereint und der Rektifikationskolonne zugeführt. Die Rektifikationskolonne wird bei ca. 1.1 bar betrieben. Am Sumpf der Rektifikationskolonne wird ein flüssiger Wasserstrom von 1.8 kg/h abgeführt. Am Kopf der Rektifikationskolonne wird ein dampfförmiger Methanolstrom von 46.2 kg/h entnommen, in einem Wärmetauscher auf 75 °C vorgewärmt und dann einem ersten Verdichter zugeführt. In diesem Verdichter wird der Strom auf 1.7 bar verdichtet. Anschließend wird der Strom aufgeteilt, ein Strom von 28.5 kg/h wird zu den Reaktionskolonnen zurückgeführt. Der Rest von 17.7 kg/h wird einer zweistufigen Verdichtung mit Zwischenkühlung zugeführt. Im Verdichter 2 wird auf 4.9 bar und 180 °C verdichtet. In der anschließenden Zwischenkühlung wird der Strom auf ca. 140 °C abgekühlt, wobei ca. 0.36 kW an Wärme über Wasser als Wärmeträgermedium abgeführt werden. Im Verdichter 3 wird der Strom schließlich auf 6.2 bar und 160 °C verdichtet. Im nachfolgenden Kondensator, der zugleich der Verdampfer der Rektifikationskolonne <300> ist, werden die ca. 5 kW an Heizleistung für die Rektifikationskolonne zur Verfügung gestellt. Dem flüssigen Methanolstrom werden 4.7 kg/h Frischmethanol zugeführt und der gemischte Strom der Rektifikationskolonne am Kopf als Rücklauf aufgegeben. Das in der Verdichterzwischenkühlung erwärmte Wärmeträgermedium wird genutzt, um Energie über die Verdampfer der beiden Reaktionskolonnen einzuspeisen. Auf diese Weise können ca. 51 % der benötigten Heizleistung über die Zwischenkühlung zur Verfügung gestellt werden. Nur die restlichen 49 % müssen mit Hilfe von Niederdruckdampf über die Verdampfer bereitgestellt werden.

**Ergebnis:** Die vorliegende Erfindung ermöglicht eine effiziente Verwendung der bei der Zwischenkühlung der Verdichterstufen ansonsten ungenutzt dissipierenden Energie.

## Patentansprüche

1. Verfahren zur Herstellung mindestens eines Alkalimetallalkoholats der Formel M_{A}OR, wobei R ein C₁ bis C₆-Kohlenwasserstoffrest ist, und wobei M_{A} ein Metall ausgewählt aus Natrium, Kalium, ist, wobei:
(a1) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend ROH und M_{A}OR entnommen wird und am oberen Ende von **RR_{A}** ein Brüdenstrom **S_{AB}** umfassend Wasser und ROH entnommen wird,
(a2) und gegebenenfalls, gleichzeitig mit und räumlich getrennt von Schritt (a1), ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohprodukt **RP_{B}** umfassend M_{B}OR, Wasser, ROH, M_{B}OH umgesetzt wird, wobei M_{B} ein Metall ausgewählt aus Natrium, Kalium ist,
wobei am unteren Ende von **RR_{B}** ein Sumpfproduktstrom **S_{BP}** umfassend ROH und M_{B}OR entnommen wird und am oberen Ende von **RR_{B}** ein Brüdenstrom **S_{BB}** umfassend Wasser und ROH entnommen wird,
(b) der Brüdenstrom **S_{AB},** und, wenn Schritt (a2) durchgeführt wird, der Brüdenstrom **S_{BB},** vermischt mit **S_{AB}** oder getrennt von **S_{AB},** in eine Rektifikationskolonne **RD_{A}** geleitet wird, und in **RD_{A}** in einen ROH umfassenden Brüdenstrom **S_{B2}** mit einem Druck **p_{B2}** und einer Temperatur **T_{B2}** am Kopf von **RD_{A}** und einen Wasserstrom **S_{W}** am Sumpf von **RD_{A}** aufgetrennt wird,
(c) mindestens ein Teil des Brüdenstroms **S_{B2}** verdichtet wird, wodurch ein gegenüber **S_{B2}** verdichteter Brüdenstrom **S_{B3}** mit einem Druck **p_{B3}** > **p_{B2}** und einer Temperatur **T_{B3}** > **T_{B2}** erhalten wird,
(d) Energie von **S_{B3}** auf mindestens einen von **RP_{A}, S_{AE1}, S_{AE2}, S_{AP}, S_{AB}** übertragen wird, und, wenn Schritt (a2) durchgeführt wird, alternativ oder zusätzlich auf mindestens einen von **RP_{B}, S_{BE1}**, **S_{BE2}, S_{BP}, S_{BB}** übertragen wird,
wodurch ein Brüdenstrom **S_{B4}** mit einem geringeren Energiegehalt als **S_{B3}** erhalten wird.

2. Verfahren nach Anspruch 1, wobei R Methyl oder Ethyl ist.

3. Verfahren nach Anspruch 1 oder 2, wobei **S_{AE2}** neben M_{A}OH mindestens eine weitere Verbindung ausgewählt aus Wasser, ROH umfasst und wobei, falls Schritt (a2) durchgeführt wird, **S_{BE2}** neben M_{B}OH mindestens eine weitere Verbindung ausgewählt aus Wasser, ROH umfasst.

4. Verfahren nach einem der Ansprüche 1 bis 3, wobei die Rektifikationskolonne **RD_{A}**, die Reaktionskolonne **RR_{A}** und, falls Schritt (a2) durchgeführt wird, die Reaktionskolonne **RR_{B}** in einem Kolonnenmantel untergebracht sind, wobei die Kolonne mindestens teilweise durch mindestens eine Trennwand und, falls Schritt (a2) durchgeführt wird, durch mindestens zwei Trennwände, unterteilt ist, wobei die mindestens eine Trennwand bzw. die mindestens zwei Trennwände bis zum Boden der Kolonne durchgezogen sind.

5. Verfahren nach einem der Ansprüche 1 bis 4, wobei 10 bis 90 Gew.-%, des Brüdenstromes **S_{B2}** als Eduktstrom **S_{AE1}** und, falls Schritt (a2) durchgeführt wird, alternativ oder zusätzlich als Eduktstrom **S_{BE1}** eingesetzt werden.

6. Verfahren nach einem der Ansprüche 1 bis 5, wobei in Schritt (d) mit **S_{B3}** mindestens einer von **RP_{A}, S_{AE1}, S_{AE2}, S_{AP}, S_{AB}** beheizt wird, und, falls Schritt (a2) durchgeführt wird, alternativ oder zusätzlich mit **S_{B3}** mindestens einer von **RP_{B}, S_{BE1}, S_{BE2}, S_{BP}, S_{BB}** beheizt wird.

7. Verfahren nach einem der Ansprüche 1 bis 6, wobei **RR_{A}** mindestens einen Verdampfer aufweist und wobei, falls Schritt (a2) durchgeführt wird, **RR_{B}** mindestens einen Verdampfer aufweist.

8. Verfahren nach Anspruch 7, wobei **RR_{A}** mindestens einen Sumpfverdampfer **V_{SA}** aufweist und wobei, falls Schritt (a2) durchgeführt wird, **RR_{B}** mindestens einen Sumpfverdampfer **V_{SB}** aufweist.

9. Verfahren nach Anspruch 8, wobei **S_{B3}** mindestens teilweise über einen Sumpfverdampfer **V_{SA}** an der Reaktionskolonne **RR_{A}** geleitet wird und **S_{AP}** mindestens teilweise über **V_{SA}** geleitet wird, so dass Energie von **S_{B3}** auf **S_{AP}** übertragen wird und, falls Schritt (a2) durchgeführt wird, alternativ oder zusätzlich **S_{B3}** mindestens teilweise über einen Sumpfverdampfer **V_{SB}** an der Reaktionskolonne **RR_{B}** geleitet wird und **S_{BP}** mindestens teilweise über **V_{SB}** geleitet wird, so dass Energie von **S_{B3}** auf **S_{BP}** übertragen wird.

10. Verfahren nach Anspruch 8, wobei **S_{B3}** einen von **RP_{A}, S_{AE1}, S_{AE2}, S_{AP}, S_{AB}, RP_{B}, S_{BE1}, S_{BE2}, S_{BP}** oder **S_{BB}** verschiedenen Wärmeträger **W₁** kontaktiert, so dass Energie von **S_{B3}** auf den mindestens einen Wärmeträger **W₁** übergeht, und **W₁** dann mindestens teilweise über einen Sumpfverdampfer **V_{SA}** an der Reaktionskolonne **RR_{A}** geleitet wird und **S_{AP}** mindestens teilweise über **V_{SA}** geleitet wird, wobei Energie von **W₁** auf **S_{AP}** übertragen wird und, falls Schritt (a2) durchgeführt wird, alternativ oder zusätzlich, bevorzugt zusätzlich, **W₁** dann mindestens teilweise über einen Sumpfverdampfer **V_{SB}** an der Reaktionskolonne **RR_{B}** geleitet wird und **S_{BP}** mindestens teilweise über **V_{SB}** geleitet wird, wobei Energie von **W₁** auf **S_{BP}** übertragen wird.

11. Verfahren nach Anspruch 10, wobei **W₁** Wasser ist.

12. Verfahren nach einem der Ansprüche 1 bis 11, wobei in einem Schritt (e) der Brüdenstrom **S_{B4}** zur Beheizung der Rektifikationskolonne **RD_{A}** eingesetzt und/oder in diese zurückgeführt wird.

13. Verfahren nach einem der Ansprüche 1 bis 12, welches kontinuierlich durchgeführt wird.

## Claims

1. Process for producing at least one alkali metal alkoxide of formula M_{A}OR, wherein R is a C₁ to C₆ hydrocarbon radical and wherein M_{A} is a metal selected from sodium, potassium, wherein:
(a1) a reactant stream **S_{AE1}** comprising ROH is reacted with a reactant stream **S_{AE2}** comprising M_{A}OH in countercurrent in a reactive rectification column **RR_{A}** to afford a crude product **RP_{A}** comprising M_{A}OR, water, ROH, M_{A}OH,
wherein a bottoms product stream **S_{AP}** comprising ROH and M_{A}OR is withdrawn at the lower end of **RR_{A}** and a vapour stream **S_{AB}** comprising water and ROH is withdrawn at the upper end of **RR_{A},**
(a2) and optionally, simultaneously with and spatially separate from step (a1), a reactant stream **S_{BE1}** comprising ROH is reacted with a reactant stream **S_{BE2}** comprising M_{B}OH in countercurrent in a reactive rectification column **RR_{B}** to afford a crude product **RP_{B}** comprising M_{B}OR, water, ROH, M_{B}OH, wherein M_{B} is a metal selected from sodium, potassium,
wherein a bottoms product stream **S_{BP}** comprising ROH and M_{B}OR is withdrawn at the lower end of **RR_{B}** and a vapour stream **S_{BB}** comprising water and ROH is withdrawn at the upper end of **RR_{B},**
(b) the vapour stream **S_{AB}** and, when step (a2) is performed, the vapour stream **S_{BB},** in admixture with **S_{AB}** or separately from **S_{AB},** is passed into a rectification column **RD_{A}** and in **RD_{A}** is separated into an ROH-comprising vapour stream **S_{B2}** having a pressure **p_{B2}** and a temperature **T_{B2}** at the top of **RD_{A}** and a water stream **Sw at** the bottom of **RD_{A},**
(c) at least a portion of the vapour stream **S_{B2}** is compressed to obtain a vapour stream **S_{B3}** compressed with respect to **S_{B2}** having a pressure **p_{B3}** > **p_{B2}** and a temperature**T_{B3}** > **T_{B2},**
(d) energy from **S_{B3}** is transferred to at least one of **RP_{A}, S_{AE1}, S_{AE2}, S_{AP}**, **S_{AB}** and, when step (a2) is performed, alternatively or additionally, transferred to at least one of **RP_{B}, S_{BE1}, S_{BE2}**, **S_{BP}, S_{BB}**,
to obtain a vapour stream **S_{B4}** having a lower energy content than **S_{B3}**.

2. Process according to Claim 1, wherein R is methyl or ethyl.

3. Process according to Claim 1 or 2, wherein **S_{AE2}** comprises not only M_{A}OH but also at least one further compound selected from water, ROH and wherein, when step (a2) is performed, **S_{BE2}** comprises not only M_{B}OH but also at least one further compound selected from water, ROH.

4. Process according to any of Claims 1 to 3, wherein the rectification column **RD_{A},** the reaction column **RR_{A}** and, when step (a2) is performed, the reaction column **RR_{B}** are accommodated in one column shell, wherein the column is at least partially subdivided by at least one dividing wall and, when step (a2) is performed, by at least two dividing walls, wherein the at least one dividing wall or the at least two dividing walls extend to the bottom of the column.

5. Process according to any of Claims 1 to 4, wherein 10% to 90% by weight of the vapour stream **S_{B2}** is employed as reactant stream **S_{AE1}** and, when step (a2) is performed, alternatively or additionally as reactant stream **S_{BE1}**.

6. Process according to any of Claims 1 to 5, wherein in step (d) **S_{B3}** is used to heat least one of **RP_{A}, S_{AE1}, S_{AE2}, S_{AP}**, **S_{AB}** and, when step (a2) is performed, alternatively or additionally, **S_{B3}** is used to heat at least one of **RP_{B}, S_{BE1}, S_{BE2}, S_{BP}**, **S_{BB}**.

7. Process according to any of Claims 1 to 6, wherein **RR_{A}** comprises at least one evaporator and wherein, when step (a2) is performed, **RR_{B}** comprises at least one evaporator.

8. Process according to Claim 7, wherein **RR_{A}** comprises at least one bottoms evaporator **V_{SA}** and wherein, when step (a2) is performed, **RR_{B}** comprises at least one bottoms evaporator **V_{SB}**.

9. Process according to Claim 8, wherein **S_{B3}** is at least partially passed through a bottoms evaporator **V_{SA}** on the reaction column **RR_{A}** and **S_{AP}** is at least partially passed through **V_{SA}** so that energy is transferred from **S_{B3}** to **S_{AP}** and, when step (a2) is performed, alternatively or in addition, **S_{B3}** is at least partially passed through a bottoms evaporator **V_{SB}** on the reaction column **RR_{B}** and **S_{BP}** is at least partially passed through **V_{SB},** so that energy is transferred from **S_{B3}** to **S_{BP}.**

10. Process according to Claim 8, wherein **S_{B3}** contacts a heat transfer medium **W₁** distinct from **RP_{A}, S_{AE1}, S_{AE2}, S_{AP}**, **S_{AB}, RP_{B}, S_{BE1}, S_{BE2}**, **S_{BP}** or **S_{BB}** so that energy is transferred from **S_{B3}** to the at least one heat transfer medium **W₁** and **W₁** is then at least partially passed through a bottoms evaporator **V_{SA}** on the reaction column **RR_{A}** and **S_{AP}** is at least partially passed through **V_{SA}** to transfer energy from **W₁** to **S_{AP}** and, when step (a2) is performed, alternatively or in addition, preferably in addition, **W₁** is then at least partially passed through a bottoms evaporator **V_{SB}** on the reaction column **RR_{B}** and **S_{BP}** is at least partially passed through **V_{SB}** to transfer energy from **W₁** to **S_{BP}.**

11. Process according to Claim 10, wherein **W₁** is water.

12. Process according to any of Claims 1 to 11, wherein the vapour stream **S_{B4}** is used for heating the rectification column **RD_{A}** and/or recycled into said column in a step (e).

13. Process according to any of Claims 1 to 12, wherein said process is performed continuously.

## Revendications

1. Procédé pour la préparation d'au moins un alcoolate de métal alcalin de formule M_{A}OR, dans laquelle R représente un radical hydrocarboné en C₁-C₆ et M_{A} représente un métal choisi parmi sodium, potassium, dans lequel :
(a1) un flux de départ S_{AE1} comprenant ROH est transformé avec un flux de départ S_{AE2} comprenant M_{A}OH à contre-courant dans une colonne de rectification réactive RR_{A} en un produit brut RP_{A} comprenant M_{A}OR, de l'eau, ROH, M_{A}OH, où, en l'extrémité inférieure de RR_{A}, est prélevé un flux de produit de fond S_{AP} comprenant ROH et M_{A}OR et, en l'extrémité supérieure de RR_{A}, est prélevé un flux de vapeur S_{AB} comprenant de l'eau et ROH,
(a2) et le cas échéant, simultanément avec l'étape (a1) et de manière spatialement séparée de celle-ci, un flux de départ S_{BE1} comprenant ROH est transformé avec un flux de départ S_{BE2} comprenant M_{B}OH à contre-courant dans une colonne de rectification réactive RR_{B} en un produit brut RP_{B} comprenant M_{B}OR, de l'eau, ROH, M_{B}OH, M_{B} étant un métal choisi parmi sodium, potassium, où, en l'extrémité inférieure de RR_{B}, est prélevé un flux de produit de fond S_{BP} comprenant ROH et M_{B}OR et, en l'extrémité supérieure de RR_{B}, est prélevé un flux de vapeur S_{BB} comprenant de l'eau et ROH,
(b) le flux de vapeur S_{AB}, et, lorsque l'étape (a2) est mise en oeuvre, le flux de vapeur S_{BB}, mélangé avec S_{AB} ou séparément de S_{AB}, est/sont conduit (s) dans une colonne de rectification RD_{A} et séparé(s) dans RD_{A} en un flux de vapeur S_{B2} comprenant ROH à une pression p_{B2} et à une température T_{B2} en tête de RD_{A} et en un flux d'eau S_{W} dans le fond de RD_{A},
(c) au moins une partie du flux de vapeur S_{B2} est comprimé, suite à quoi on obtient un flux de vapeur S_{B3} comprimé par rapport à S_{B2} à une pression p_{B3} > p_{B2} et à une température T_{B3} > T_{B2},
(d) de l'énergie est transférée de S_{B3} sur au moins l'un parmi RP_{A}, S_{AE1}, S_{AE2}, S_{AB}, S_{AB} et, lorsque l'étape (a2) est mise en oeuvre, en variante ou en plus à au moins l'un parmi RP_{B}, S_{BE1}, S_{BE2}, S_{BB}, S_{BB},
suite à quoi un flux de vapeur S_{B4} présentant une teneur en énergie inférieure à celle dans S_{B3} est obtenu.

2. Procédé selon la revendication 1, dans lequel R représente méthyle ou éthyle.

3. Procédé selon la revendication 1 ou 2, dans lequel S_{AE2} comprend, outre M_{A}OH, au moins un autre composé choisi parmi l'eau, ROH et, si l'étape (a2) est mise en oeuvre, S_{BE2} comprend, outre M_{B}OH, au moins un autre composé choisi parmi l'eau, ROH.

4. Procédé selon l'une des revendications 1 à 3, dans lequel la colonne de rectification RD_{A}, la colonne de réaction RR_{A} et, si l'étape (a2) est mise en oeuvre, la colonne de réaction RR_{B} sont logées dans une enveloppe de colonne, la colonne étant subdivisée au moins partiellement par au moins une paroi de séparation et, si l'étape (a2) est mise en oeuvre, par au moins deux parois de séparation, ladite au moins une paroi de séparation ou, selon le cas, lesdites au moins deux parois de séparation allant jusqu'au fond de la colonne.

5. Procédé selon l'une des revendications 1 à 4, dans lequel 10 à 90% en poids du flux de vapeur S_{B2} sont utilisés comme flux de départ S_{AE1} et, si l'étape (a2) est mise en oeuvre, en variante ou en plus comme flux de départ S_{BE1}.

6. Procédé selon l'une des revendications 1 à 5, dans lequel, dans l'étape (d), S_{B3} chauffe au moins l'un parmi RP_{A}**,** S_{AE1}, S_{AE2}, S_{AB}, S_{AB} et, si l'étape (a2) est mise en oeuvre, en variante ou en plus, S_{B3} chauffe au moins l'un parmi RP_{B}**,** S_{BE1}, S_{BE2}, S_{BP}**,** S_{BB}.

7. Procédé selon l'une des revendications 1 à 6, dans lequel RR_{A} présente au moins un évaporateur et, si l'étape (a2) est mise en oeuvre, RR_{B} présente au moins un évaporateur.

8. Procédé selon la revendication 7, dans lequel RR_{A} présente au moins un évaporateur de fond V_{SA} et, si l'étape (a2) est mise en oeuvre, RR_{B} présente au moins un évaporateur de fond V_{SB}.

9. Procédé selon la revendication 8, dans lequel S_{B3} est guidé au moins partiellement par l'intermédiaire d'un évaporateur de fond V_{SA} vers la colonne de réaction RR_{A} et S_{AP} est guidé au moins partiellement par l'intermédiaire de V_{SA}, de telle sorte que l'énergie est transférée de S_{B3} à S_{AP} et, si l'étape (a2) est mise en oeuvre, en variante ou en plus, S_{B3} est guidé au moins partiellement par l'intermédiaire d'un évaporateur de fond V_{SB} vers la colonne de réaction RR_{B} et S_{BP} est guidé au moins partiellement par l'intermédiaire de V_{SB}, de telle sorte que de l'énergie est transférée de S_{B3} à S_{BP}**.**

10. Procédé selon la revendication 8, dans lequel S_{B3} entre en contact avec un fluide caloporteur W₁ différent de RP_{A}**,** S_{AE1}, S_{AE2}, S_{AP}, S_{AB}, RP_{B}**,** S_{BE1}, S_{BE2}, S_{BP} ou S_{BB}, de telle sorte que l'énergie passe de S_{B3} audit au moins un fluide caloporteur W₁ et W₁ est ensuite guidé au moins partiellement par l'intermédiaire d'un évaporateur de fond V_{SA} vers la colonne de réaction RR_{A} et S_{AP} est guidé au moins partiellement par l'intermédiaire de V_{SA}, l'énergie étant transférée de W₁ à S_{AP} et, si l'étape (a2) est mise en oeuvre, en variante ou en plus, de préférence en plus, W₁ est alors guidé au moins partiellement par l'intermédiaire d'un évaporateur de fond V_{SB} vers la colonne de réaction RR_{B} et S_{BP} est guidé au moins partiellement par l'intermédiaire de V_{SB}, l'énergie étant transférée de W₁ à S_{BP}**.**

11. Procédé selon la revendication 10, dans lequel W₁ est de l'eau.

12. Procédé selon l'une des revendications 1 à 11, dans lequel, dans une étape (e), le flux de vapeur S_{B4} est utilisé pour chauffer la colonne de rectification RD_{A} et/ou recyclé dans celle-ci.

13. Procédé selon l'une ou plusieurs des revendications 1 à 12, qui est mis en oeuvre en continu.
